(19) Europäisches Patentamt
European Patent Office
Office européen des brevets

(11) EP 3 274 721 B1

(12) **EUROPEAN PATENT SPECIFICATION**

(45) Date of publication and mention of the grant of the patent:
**25.11.2020 Bulletin 2020/48**

(21) Application number: **16710459.5**

(22) Date of filing: **18.03.2016**

(51) Int Cl.:
***G01N 33/68*** *(2006.01)* ***C07K 7/06*** *(2006.01)*
***C07K 7/08*** *(2006.01)*

(86) International application number:
**PCT/EP2016/055961**

(87) International publication number:
**WO 2016/150853 (29.09.2016 Gazette 2016/39)**

(54) **RETENTION TIME STANDARD**

RETENTIONSZEITSTANDARD

ÉTALON DE TEMPS DE RÉTENTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2015 GB 201504820**

(43) Date of publication of application:
**31.01.2018 Bulletin 2018/05**

(73) Proprietor: **Polyquant GmbH**
**93077 Bad Abbach (DE)**

(72) Inventors:
- **EYERS, Claire, E.**
**Meols**
**Wirral CH47 7AP (GB)**
- **HOLMAN, Stephen, W.**
**Manchester M1 7EP (GB)**

(74) Representative: **PATERIS Patentanwälte PartmbB**
**Postfach 33 07 11**
**80067 München (DE)**

(56) References cited:
**EP-A1- 1 983 344**

- **OLEG V. KROKHIN ET AL: "Peptide Retention Standards and Hydrophobicity Indexes in Reversed-Phase High-Performance Liquid Chromatography of Peptides", ANALYTICAL CHEMISTRY, vol. 81, no. 22, 15 November 2009 (2009-11-15), pages 9522-9530, XP055139121, ISSN: 0003-2700, DOI: 10.1021/ac9016693**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 274 721 B1

SEQUENCE LISTING

<110> Polyquant GmbH

<120> Retention time standard

<130> P30052

<150> GB1504820.0
<151> 2015-03-23

<160> 36

<170> BiSSAP 1.3

<210> 1
<211> 12
<212> PRT
<213> Artificial Sequence

<220>
<223> reference peptide

<400> 1

```
Val Thr Ala Ser Gly Asp Asp Ser Pro Ser Gly Lys
1               5                   10
```

<210> 2
<211> 9
<212> PRT
<213> Artificial Sequence

<220>
<223> reference peptide

<400> 2

```
Ala Leu Ala Glu Asp Glu Gly Ala Lys
1               5
```

<210> 3
<211> 11
<212> PRT
<213> Artificial Sequence

<220>
<223> reference peptide

<400> 3

```
Ala Ser Ala Asp Leu Gln Pro Asp Ser Gln Lys
1               5                   10
```

<210> 4
<211> 11
<212> PRT
<213> Artificial Sequence

<220>
<223> reference peptide

<400> 4

Ser Ser Tyr Val Gly Asp Glu Ala Ser Ser Lys
1 5 10

<210> 5
<211> 15
<212> PRT
<213> Artificial Sequence

<220>
<223> reference peptide

<400> 5

Ala Ala Ala Pro Glu Pro Glu Thr Glu Thr Glu Thr Ser Ser Lys
1 5 10 15

<210> 6
<211> 8
<212> PRT
<213> Artificial Sequence

<220>
<223> reference peptide

<400> 6

Ile Val Pro Glu Pro Gln Pro Lys
1 5

<210> 7
<211> 10
<212> PRT
<213> Artificial Sequence

<220>
<223> reference peptide

<400> 7

Gly Ala Ile Glu Thr Glu Pro Ala Val Lys
1 5 10

<210> 8
<211> 13
<212> PRT
<213> Artificial Sequence

<220>
<223> reference peptide

<400> 8

Phe His Pro Gly Thr Asp Glu Gly Asp Tyr Gln Val Lys
1 5 10

<210> 9
<211> 8
<212> PRT
<213> Artificial Sequence

<220>
<223> reference peptide

<400> 9

```
Val Gly Tyr Asp Leu Pro Gly Lys
1               5
```

<210> 10
<211> 12
<212> PRT
<213> Artificial Sequence

<220>
<223> reference peptide

<400> 10

```
Ser Ala Gly Gly Ala Phe Gly Pro Glu Leu Ser Lys
1               5                   10
```

<210> 11
<211> 13
<212> PRT
<213> Artificial Sequence

<220>
<223> reference peptide

<400> 11

```
Thr Ala Ser Glu Phe Glu Ser Ala Ile Asp Ala Gln Lys
1               5                   10
```

<210> 12
<211> 13
<212> PRT
<213> Artificial Sequence

<220>
<223> reference peptide

<400> 12

```
Gly Val Asn Asp Asn Glu Glu Gly Phe Phe Ser Ala Lys
1               5                   10
```

<210> 13
<211> 11
<212> PRT

<213> Artificial Sequence

<220>
<223> reference peptide

<400> 13

Val Gly Leu Phe Ala Gly Ala Gly Val Gly Lys
1 5 10

<210> 14
<211> 10
<212> PRT
<213> Artificial Sequence

<220>
<223> reference peptide

<400> 14

Thr Gln Leu Ile Asp Val Glu Ile Ala Lys
1 5 10

<210> 15
<211> 9
<212> PRT
<213> Artificial Sequence

<220>
<223> reference peptide

<400> 15

Leu Thr Val Leu Glu Ser Leu Ser Lys
1 5

<210> 16
<211> 14
<212> PRT
<213> Artificial Sequence

<220>
<223> reference peptide

<400> 16

Leu Ala Pro Asp Leu Ile Val Val Ala Gln Thr Gly Gly Lys
1 5 10

<210> 17
<211> 9
<212> PRT
<213> Artificial Sequence

<220>

<223> reference peptide

<400> 17

Leu Thr Ile Ala Pro Ala Leu Leu Lys
1 5

<210> 18
<211> 14
<212> PRT
<213> Artificial Sequence

<220>
<223> reference peptide

<400> 18

Ile Leu Thr Asp Ile Val Gly Pro Glu Ala Pro Leu Val Lys
1 5 10

<210> 19
<211> 8
<212> PRT
<213> Artificial Sequence

<220>
<223> reference peptide

<400> 19

Leu Thr Ile Glu Glu Phe Leu Lys
1 5

<210> 20
<211> 19
<212> PRT
<213> Artificial Sequence

<220>
<223> reference peptide

<400> 20

Thr Ser Ala Glu Ser Ile Leu Thr Thr Gly Pro Val Val Pro Val Ile
1 5 10 15
Val Val Lys

<210> 21
<211> 23
<212> PRT
<213> Artificial Sequence

<220>
<223> reference peptide

<400> 21

```
Ile Ser Ser Ile Asp Leu Ser Val Leu Asp Ser Pro Leu Ile Pro Ser
1               5                   10                  15
Ala Thr Thr Gly Thr Ser Lys
            20
```

<210> 22
<211> 31
<212> PRT
<213> Artificial Sequence

<220>
<223> reference peptide

<400> 22

```
Ala Gly Leu Glu Phe Gly Thr Thr Pro Glu Gln Pro Glu Glu Thr Pro
1               5                   10                  15
Leu Asp Asp Leu Ala Glu Thr Asp Phe Gln Thr Phe Ser Gly Lys
            20                  25                  30
```

<210> 23
<211> 12
<212> PRT
<213> Artificial Sequence

<220>
<223> reference peptide

<400> 23

```
Val Val Ser Leu Pro Asp Phe Phe Thr Phe Ser Lys
1               5                   10
```

<210> 24
<211> 17
<212> PRT
<213> Artificial Sequence

<220>
<223> reference peptide

<400> 24

```
Ala Val Thr Thr Leu Ala Glu Ala Val Val Ala Ala Thr Leu Gly Pro
1               5                   10                  15
Lys
```

<210> 25
<211> 13
<212> PRT
<213> Artificial Sequence

<220>
<223> reference peptide

<400> 25

Ile Ala Phe Phe Glu Ser Ser Phe Leu Ser Tyr Leu Lys
1 5 10

<210> 26
<211> 19
<212> PRT
<213> Artificial Sequence

<220>
<223> reference peptide

<400> 26

Ser Ser Ile Pro Val Phe Gly Val Asp Ala Leu Pro Glu Ala Leu Ala
1 5 10 15
Leu Val Lys

<210> 27
<211> 18
<212> PRT
<213> Artificial Sequence

<220>
<223> reference peptide

<400> 27

Phe Leu Ser Ser Pro Phe Ala Val Ala Glu Val Phe Thr Gly Ile Val
1 5 10 15
Gly Lys

<210> 28
<211> 4
<212> PRT
<213> Artificial Sequence

<220>
<223> C-terminal peptide

<400> 28

Met Gly Thr Lys
1

<210> 29
<211> 12
<212> PRT
<213> Artificial Sequence

<220>
<223> N-terminal peptide

<400> 29

```
Leu Ala Ala Ala Leu Glu His His His His His His
1               5                   10
```

<210> 30
<211> 23
<212> PRT
<213> Artificial Sequence

<220>
<223> artificial peptide

<400> 30

```
Ala Ala Ala Pro Glu Pro Glu Thr Glu Thr Glu Thr Ser Ser Lys Ile
1               5                   10                  15
Val Pro Glu Pro Gln Pro Lys
            20
```

<210> 31
<211> 1146
<212> DNA
<213> Artificial Sequence

<220>
<223> Expression construct

<400> 31

```
catatgggta ccaaagtaac cgcgtctggc gatgattctc cgagcggcaa agccctggct      60
gaagatgaag gtgcgaaagc aagcgcagac ctgcagccgg attctcagaa aagctcttac     120
gttggcgatg aagcctcttc caaagcagcc gctccagaac cagagaccga aactgagacc     180
agctctaaaa tcgttccgga accgcagccg aaaggtgcta tcgaaaccga accagcggtg     240
aaattccacc cgggtaccga tgaaggcgat taccaggtga aagttggtta tgacctgccg     300
ggtaaatctg caggtggcgc ttttggtccg gaactgagca aaactgcgtc cgaattcgag     360
tctgcgattg acgctcagaa aggtgtcaac gacaacgagg aaggtttctt ctccgcaaaa     420
gttggcctgt ttgcaggcgc tggtgttggc aaaacccagc tgatcgacgt tgagatcgcg     480
aaactgacgg tcctggaatc cctgtctaaa ctggccccag acctgattgt tgtagcgcag     540
actggcggta aactcacgat tgcaccagcg ctgctgaaaa tcctgaccga tatcgtaggt     600
ccggaagctc cgctggtgaa actgacgatt gaggaattcc tgaaaacctc cgcggaatct     660
atcctcacga ctggtccggt cgtgccagtt attgttgtca aaatctcttc catcgacctg     720
agcgtgctgg attctccact gattccgagc gcaactactg gcacctctaa agctggtctg     780
gaattcggca ccactccgga acagccagaa gagactccgc tcgacgacct ggcagaaacc     840
gactttcaga ccttcagcgg caaagttgtg tccctgccgg acttctttac cttctccaaa     900
gcggtaacga ccctggctga agcagttgta gctgcaaccc tgggtccgaa aatcgccttc     960
ttcgagtcct ctttcctgtc ttacctgaaa agctctatcc cagtcttcgg tgttgatgca    1020
ctgccagaag ctctggcact ggtgaaattc ctgtcctctc cgtttgcggt ggctgaagtc    1080
tttaccggca tcgtaggtaa actggctgcc gcactggaac accatcacca tcaccactga    1140
ggatcc                                                               1146
```

<210> 32
<211> 378
<212> PRT
<213> Artificial Sequence

<220>
<223> artificial polypeptide

<400> 32

```
Met Gly Thr Lys Val Thr Ala Ser Gly Asp Asp Ser Pro Ser Gly Lys
1               5                   10                  15
Ala Leu Ala Glu Asp Glu Gly Ala Lys Ala Ser Ala Asp Leu Gln Pro
            20                  25                  30
Asp Ser Gln Lys Ser Ser Tyr Val Gly Asp Glu Ala Ser Ser Lys Ala
        35                  40                  45
Ala Ala Pro Glu Pro Glu Thr Glu Thr Glu Thr Ser Ser Lys Ile Val
    50                  55                  60
Pro Glu Pro Gln Pro Lys Gly Ala Ile Glu Thr Glu Pro Ala Val Lys
65                  70                  75                  80
Phe His Pro Gly Thr Asp Glu Gly Asp Tyr Gln Val Lys Val Gly Tyr
                85                  90                  95
Asp Leu Pro Gly Lys Ser Ala Gly Gly Ala Phe Gly Pro Glu Leu Ser
            100                 105                 110
Lys Thr Ala Ser Glu Phe Glu Ser Ala Ile Asp Ala Gln Lys Gly Val
        115                 120                 125
Asn Asp Asn Glu Glu Gly Phe Phe Ser Ala Lys Val Gly Leu Phe Ala
    130                 135                 140
Gly Ala Gly Val Gly Lys Thr Gln Leu Ile Asp Val Glu Ile Ala Lys
145                 150                 155                 160
Leu Thr Val Leu Glu Ser Leu Ser Lys Leu Ala Pro Asp Leu Ile Val
                165                 170                 175
Val Ala Gln Thr Gly Gly Lys Leu Thr Ile Ala Pro Ala Leu Leu Lys
            180                 185                 190
Ile Leu Thr Asp Ile Val Gly Pro Glu Ala Pro Leu Val Lys Leu Thr
        195                 200                 205
Ile Glu Glu Phe Leu Lys Thr Ser Ala Glu Ser Ile Leu Thr Thr Gly
    210                 215                 220
Pro Val Val Pro Val Ile Val Val Lys Ile Ser Ser Ile Asp Leu Ser
225                 230                 235                 240
Val Leu Asp Ser Pro Leu Ile Pro Ser Ala Thr Thr Gly Thr Ser Lys
                245                 250                 255
Ala Gly Leu Glu Phe Gly Thr Thr Pro Glu Gln Pro Glu Glu Thr Pro
            260                 265                 270
Leu Asp Asp Leu Ala Glu Thr Asp Phe Gln Thr Phe Ser Gly Lys Val
        275                 280                 285
Val Ser Leu Pro Asp Phe Phe Thr Phe Ser Lys Ala Val Thr Thr Leu
    290                 295                 300
Ala Glu Ala Val Val Ala Ala Thr Leu Gly Pro Lys Ile Ala Phe Phe
305                 310                 315                 320
Glu Ser Ser Phe Leu Ser Tyr Leu Lys Ser Ser Ile Pro Val Phe Gly
                325                 330                 335
Val Asp Ala Leu Pro Glu Ala Leu Ala Leu Val Lys Phe Leu Ser Ser
            340                 345                 350
Pro Phe Ala Val Ala Glu Val Phe Thr Gly Ile Val Gly Lys Leu Ala
        355                 360                 365
Ala Ala Leu Glu His His His His His His
    370                 375
```

<210> 33
<211> 1146
<212> DNA
<213> Artificial Sequence

<220>
<223> Expression construct

<400> 33

```
catatgggca cgaaaggcgc tatcgagacc gaaccagcag tcaaaatctc ttccattgat      60
ctgtctgtac tggactctcc gctgattcca tctgcgacga ctggcacctc caaaatcctg     120
accgacattg ttggtccgga agcaccactg gttaaattcc atccgggcac ggatgagggt     180
gactaccagg tcaaatctgc tggtggtgct tttggcccgg aactgtctaa actgacgatc     240
gcaccagcgc tgctgaaaat cgccttcttt gaatcctctt tcctgtctta tctgaaactg     300
accatcgagg aattcctgaa aacttctgcg gaaagcattc tgaccactgg cccagtggtt     360
ccggttatcg tagtgaaact caccgtcctg gaaagcctgt ctaaaggtgt aaacgataac     420
gaagaaggct tttctccgc gaaatctagc attccggtat tcggtgtgga tgcgctgccg     480
gaagccctgg cactggtgaa aacccagctg attgacgttg agatcgctaa agccctggct     540
gaggacgaag gtgcgaaagt ggttagcctg ccggatttct tcaccttctc taaagcagtt     600
acgaccctcg cggaagccgt ggtagctgcc accctgggcc cgaaatcctc ctacgttggt     660
gatgaagctt ctagcaaagc gtctgcagac ctgcagccgg actctcagaa agtcactgcc     720
tccggcgatg actccccatc cggcaaaacc gcttctgagt ttgaaagcgc tatcgatgcg     780
cagaaagcag gtctcgagtt tggtactacc ccagaacagc cggaagagac cccactggat     840
gacctggcgg aaaccgactt ccagaccttc agcggtaaac tggcgccgga cctgatcgtt     900
gtggcacaga ctggtggcaa aatcgttccg gaaccgcagc cgaaagcagc ggctccggaa     960
ccggaaactg aaaccgaaac ctcttccaaa gttggtctgt ttgcgggtgc tggtgttggc    1020
aaattcctga gctctccatt cgcagtggcc gaagtattca ctggtatcgt gggtaaagtt    1080
ggctacgatc tgccaggtaa actggcagcg gctctggaac accaccatca ccatcactga    1140
ggatcc                                                               1146
```

<210> 34
<211> 378
<212> PRT
<213> Artificial Sequence

<220>
<223> Artificial polypeptide

<400> 34

```
Met Gly Thr Lys Gly Ala Ile Glu Thr Glu Pro Ala Val Lys Ile Ser
1               5                   10                  15
Ser Ile Asp Leu Ser Val Leu Asp Ser Pro Leu Ile Pro Ser Ala Thr
            20                  25                  30
Thr Gly Thr Ser Lys Ile Leu Thr Asp Ile Val Gly Pro Glu Ala Pro
        35                  40                  45
Leu Val Lys Phe His Pro Gly Thr Asp Glu Gly Asp Tyr Gln Val Lys
    50                  55                  60
Ser Ala Gly Gly Ala Phe Gly Pro Glu Leu Ser Lys Leu Thr Ile Ala
65                  70                  75                  80
Pro Ala Leu Leu Lys Ile Ala Phe Phe Glu Ser Ser Phe Leu Ser Tyr


                85                  90                  95
Leu Lys Leu Thr Ile Glu Glu Phe Leu Lys Thr Ser Ala Glu Ser Ile
            100                 105                 110
Leu Thr Thr Gly Pro Val Val Pro Val Ile Val Val Lys Leu Thr Val
        115                 120                 125
Leu Glu Ser Leu Ser Lys Gly Val Asn Asp Asn Glu Glu Gly Phe Phe
    130                 135                 140
Ser Ala Lys Ser Ser Ile Pro Val Phe Gly Val Asp Ala Leu Pro Glu
145                 150                 155                 160
Ala Leu Ala Leu Val Lys Thr Gln Leu Ile Asp Val Glu Ile Ala Lys
                165                 170                 175
Ala Leu Ala Glu Asp Glu Gly Ala Lys Val Val Ser Leu Pro Asp Phe
            180                 185                 190
Phe Thr Phe Ser Lys Ala Val Thr Thr Leu Ala Glu Ala Val Val Ala
        195                 200                 205
Ala Thr Leu Gly Pro Lys Ser Ser Tyr Val Gly Asp Glu Ala Ser Ser
    210                 215                 220
Lys Ala Ser Ala Asp Leu Gln Pro Asp Ser Gln Lys Val Thr Ala Ser
225                 230                 235                 240
Gly Asp Asp Ser Pro Ser Gly Lys Thr Ala Ser Glu Phe Glu Ser Ala
                245                 250                 255
Ile Asp Ala Gln Lys Ala Gly Leu Glu Phe Gly Thr Thr Pro Glu Gln
            260                 265                 270
Pro Glu Glu Thr Pro Leu Asp Asp Leu Ala Glu Thr Asp Phe Gln Thr
        275                 280                 285
Phe Ser Gly Lys Leu Ala Pro Asp Leu Ile Val Val Ala Gln Thr Gly
    290                 295                 300
Gly Lys Ile Val Pro Glu Pro Gln Pro Lys Ala Ala Ala Pro Glu Pro
305                 310                 315                 320
Glu Thr Glu Thr Glu Thr Ser Ser Lys Val Gly Leu Phe Ala Gly Ala
                325                 330                 335
Gly Val Gly Lys Phe Leu Ser Ser Pro Phe Ala Val Ala Glu Val Phe
            340                 345                 350
Thr Gly Ile Val Gly Lys Val Gly Tyr Asp Leu Pro Gly Lys Leu Ala
        355                 360                 365
Ala Ala Leu Glu His His His His His His
    370                 375
```

<210> 35
<211> 1146
<212> DNA
<213> Artificial Sequence

<220>
<223> Expression construct

<400> 35

```
catatgggca ccaaactgac catcgaggaa ttcctgaaat ttctgagctc tccgtttgct      60
gttgctgaag ttttcaccgg tatcgtaggt aaagcagcgg caccggaacc ggagaccgaa     120
accgaaacct cctctaaaat cctgactgat atcgtgggcc cggaagctcc actggtaaaa     180
ggtgccattg aaaccgaacc agcggtcaaa atcgtgccgg aaccgcagcc gaaactggct     240
ccggacctga tcgttgtggc acagacgggc ggtaaagctc tggctgaaga tgaaggtgcg     300
aaaactgcga gcgagttcga atctgccatc gatgcgcaga aaggtgtgaa cgataacgaa     360
gagggtttct tctctgccaa actgaccatt gcaccggcgc tgctgaaagc tggcctcgaa     420
ttcggtacga ccccagaaca gccagaagag actccactgg atgacctggc tgaaaccgat     480

ttccagacgt ttagcggtaa atccgcgggt ggtgcgtttg gcccagaact gagcaaaagc     540
agctacgtgg gtgacgaagc gtcctctaaa gcgtccgcgg acctgcagcc ggactcccag     600
aaactgaccg ttctggaatc tctgtccaaa gcggttacta ccctggctga ggcagttgta     660
gcagccacgc tgggtccgaa atttcaccca ggtactgatg aaggtgatta ccaggtgaaa     720
gttggctatg atctcccagg taaaatttct tccatcgacc tgtctgtcct ggactctccg     780
ctcattccga gcgcaaccac tggcacctct aaagtcgttt ccctgccgga cttcttcacc     840
ttctccaaaa cctctgctga atctatcctg acgactggcc cggtggttcc ggtaatcgtc     900
gtgaaaattg cctttttcga aagctctttc ctgtcctacc tgaaatctag catcccagtt     960
ttcggtgttg acgcactgcc ggaagcactg gctctggtga aaactcagct gatcgacgtt    1020
gagattgcca aagtaaccgc gtctggcgat gactctccgt ctggcaaagt aggcctgttc    1080
gccggcgcag gtgtaggcaa actggcggca gctctggagc accaccatca tcaccactaa    1140
ggatcc                                                                1146
```

<210> 36
<211> 378
<212> PRT
<213> Artificial Sequence

<220>
<223> Artificial polypeptide

<400> 36

```
Met Gly Thr Lys Leu Thr Ile Glu Glu Phe Leu Lys Phe Leu Ser Ser
1               5                   10                  15
Pro Phe Ala Val Ala Glu Val Phe Thr Gly Ile Val Gly Lys Ala Ala
            20                  25                  30
Ala Pro Glu Pro Glu Thr Glu Thr Glu Thr Ser Ser Lys Ile Leu Thr
        35                  40                  45
Asp Ile Val Gly Pro Glu Ala Pro Leu Val Lys Gly Ala Ile Glu Thr
    50                  55                  60
Glu Pro Ala Val Lys Ile Val Pro Glu Pro Gln Pro Lys Leu Ala Pro
65                  70                  75                  80
Asp Leu Ile Val Val Ala Gln Thr Gly Gly Lys Ala Leu Ala Glu Asp
                85                  90                  95
Glu Gly Ala Lys Thr Ala Ser Glu Phe Glu Ser Ala Ile Asp Ala Gln
            100                 105                 110
Lys Gly Val Asn Asp Asn Glu Glu Gly Phe Phe Ser Ala Lys Leu Thr
        115                 120                 125
Ile Ala Pro Ala Leu Leu Lys Ala Gly Leu Glu Phe Gly Thr Thr Pro
    130                 135                 140
Glu Gln Pro Glu Glu Thr Pro Leu Asp Asp Leu Ala Glu Thr Asp Phe
145                 150                 155                 160
Gln Thr Phe Ser Gly Lys Ser Ala Gly Gly Ala Phe Gly Pro Glu Leu
                165                 170                 175
Ser Lys Ser Ser Tyr Val Gly Asp Glu Ala Ser Ser Lys Ala Ser Ala
            180                 185                 190
Asp Leu Gln Pro Asp Ser Gln Lys Leu Thr Val Leu Glu Ser Leu Ser
        195                 200                 205
Lys Ala Val Thr Thr Leu Ala Glu Ala Val Val Ala Ala Thr Leu Gly
    210                 215                 220
Pro Lys Phe His Pro Gly Thr Asp Glu Gly Asp Tyr Gln Val Lys Val


225                 230                 235                 240
Gly Tyr Asp Leu Pro Gly Lys Ile Ser Ser Ile Asp Leu Ser Val Leu
                245                 250                 255
Asp Ser Pro Leu Ile Pro Ser Ala Thr Thr Gly Thr Ser Lys Val Val
            260                 265                 270
Ser Leu Pro Asp Phe Phe Thr Phe Ser Lys Thr Ser Ala Glu Ser Ile
        275                 280                 285
Leu Thr Thr Gly Pro Val Val Pro Val Ile Val Val Lys Ile Ala Phe
    290                 295                 300
Phe Glu Ser Ser Phe Leu Ser Tyr Leu Lys Ser Ser Ile Pro Val Phe
305                 310                 315                 320
Gly Val Asp Ala Leu Pro Glu Ala Leu Ala Leu Val Lys Thr Gln Leu
                325                 330                 335
Ile Asp Val Glu Ile Ala Lys Val Thr Ala Ser Gly Asp Asp Ser Pro
            340                 345                 350
Ser Gly Lys Val Gly Leu Phe Ala Gly Ala Gly Val Gly Lys Leu Ala
        355                 360                 365
Ala Ala Leu Glu His His His His His His
    370                 375
```

## Description

### Field of the invention

The invention relates to a composition as well as a polypeptide comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids. The invention further relates to the use of at least 8 R-peptides for reverse-phase liquid chromatography and selected reaction monitoring. Furthermore, the invention relates to a method for monitoring reverse-phase chromatography, comprising the steps providing at least 8 R-peptides, applying the R-peptides to a chromatography column, and determining the retention time of each peptide. Furthermore, the invention relates to a method for estimating a retention time of a target peptide during a liquid chromatography run.

### Background of the invention

For analyzing molecules of biological origin, in particular proteins, mass spectrometric techniques have become particularly important. For subjecting biomolecules to mass spectrometric analysis, however, they need to be isolated and purified from an original sample. In this respect reverse-phase chromatography has become the most widely used technique, eventually combined with other techniques such as gel filtration and ion exchange chromatography. For studying and analyzing biomolecules, in particular in complex samples, optimal instrument performance is paramount to ensure that the highest quality data is acquired and maximum information obtained from each analysis. Typical practice is to analyze a reliable, well-characterized standard sample that allows the analyst to monitor instrumental performance with respect to relevant parameters of interest e.g. chromatographic peak width, mass spectrometric signal response and/or protein sequence coverage.

Moreover, quality control standards enable instrument performance to be monitored longitudinally, expedite trouble shooting to maximize uptime and allow for the comparison between different experimental conditions and instruments. When using reverse-phase liquid chromatography (LC) in combination with mass spectrometry for proteomics analysis, one key parameter in the assessment of instrument performance is the retention time of peptides on the chromatography column. For example, peptide retention time has been used in many studies as an orthogonal identification criterion alongside product ion mass spectra to filter out false positive identifications and improve peptide and protein identification rates. This was typically achieved through the use of predictive models developed by machine learning algorithms (Moruz et al., 2012). Predictive programs based on fundamental physicochemical phenomena related to peptide chromatographic behaviour have also been reported (Krokhin, 2006). However, these models have been demonstrated to generally only perform well under the specific conditions for which they were developed for. In consequence, they cannot necessarily be applied to any individual liquid chromatography-mass spectrometry setup, such that empirical determination of retention time is often preferred. Therefore, approaches have been made to provide retention time standards. For example, a set of stable isotope labelled peptides was described by Burkhart et al. (2011), which were designed to test several aspects of instrument performance, including high performance liquid chromatography (HPLC) quality control. An alternative approach was suggested by Mirzaei et al. (2009), using halogenated peptides. A yet further approach was presented by Krokhin and Spicer (2009), based on a six peptide standard alongside a "hydrophobicity index", that was defined as the percentage of acetonitrile required to give a retention factor of 10 for each peptide under isocratic elution conditions. However, although these standards have been found useful for specific approaches, they usually provide only limited information regarding the quality and stability of an entire liquid chromatography run. Moreover, their use for newly developed and more sophisticated mass spectrometry analysis, such as scheduled selected reaction monitoring (SRM), are limited.

Therefore, novel retention time standards are needed, which provide a stringent and reliable quality control of reverse-phase liquid chromatography.

### Summary of the invention

In a first aspect, the invention relates to a composition comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

In a further aspect, the invention relates to a polypeptide comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

In a further aspect, the invention relates to a kit comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

In a further aspect, the invention relates to the use of at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, for reverse-phase liquid chromatography, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

In a further aspect, the invention relates to the use of at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, for selected reaction monitoring (SRM), wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

In a further aspect, the invention relates to a method for monitoring reverse-phase chromatography, comprising the steps providing at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, applying the R-peptides as single peptides to a chromatography column, and determining the retention time of each peptide, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

In a further aspect, the invention relates to a method for estimating a retention time of a target peptide during a liquid chromatography run, comprising the steps providing at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, adding the peptides to a sample comprising the target peptide, applying the sample to a chromatography column, determining the retention times of the R-peptides, and

estimating the retention time of the target peptide according to the determined retention times of the R-peptides, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

**Brief description of the drawings**

Figure 1 shows the structure of RePLiCal with cleavage sites for Lys-C and trypsin denoted by horizontal black lines. SEQ ID NO.: 22 was observed as $[M+3H]^{3+}$ species and SEQ ID NO.: 8 was observed as $[M+2H]^{2+}$ and $[M+3H]^{3+}$ species, whereas all remaining peptides were observed as $[M+2H]^{2+}$ species. The C- and *N*-terminal peptides (SEQ ID NOs.: 28 and 29) were not used for retention time (RT) standardization.

Figure 2 shows nLC-nESI-SRM-MS chromatograms acquired from Lys-C and trypsin digest of RePLiCal demonstrating that the same set of peptides are generated with good comparability upon proteolysis using the two proteases. Chromatographic profiles were generated using Waters MassLynx software including smoothing.

Figure 3 shows a comparison of chromatograms on 30 min LC gradient (3 to 40 % 0.1 % formic acid in MeCN) with nESI-SRM-MS data acquisition for RePLiCal and three commercially available retention time standards. The numerical annotations represent the elution order as provided by the manufacture. Peptides 1 and 2 from the mass spectrometry (MS) RT calibration mix (Sigma) were not observed.

Figure 4 shows the comparison of predicted retention times (RT) of RePLiCal peptides using BioLCCC and average experimentally determined values on a 30 min gradient (3 to 40 % 0.1 % formic acid in MeCN).

Figure 5 shows the comparison of the retention times (RT) of RePLiCal peptides on different length LC gradients (3 to 40 % 0.1 % formic acid in MeCN). Error bars represent +/- 2 standard deviations.

Figure 6 shows the comparison of the retention times (RT) of RePLiCal peptides on two different nano LC instruments (Thermo RSLC (A) and Waters nano ACQUITY (B)) using two different LC gradients (3 to 40 % 0.1 % formic acid in MeCN). Error bars represent +/- 2 standard deviations.

Figure 7 shows a comparison of trapping column performance using the intensities of the three earliest eluting peptides in RePLiCal.

Figure 8 shows an nLC-nESI-SRM-MS chromatogram of 5 fmol of RePLiCal spiked into 1 $\mu$g of a whole cell yeast lysate tryptic digest separated on a 30 min gradient (3 to 40 % 0.1 % formic acid in MeCN).

Figure 9 (A-D) shows the comparison of the retention times of RePLiCal peptides using 0.1 % formic acid and 0.5 % acetic acid as the ion-pairing agent for different LC gradient length (3 to 40 % in MeCN). Error bars represent +/- 2 standard deviations.

**Detailed description of the invention**

In a first aspect is disclosed herein a composition comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids. In particular, the invention relates to said composition, wherein each variant differs from the original peptide in 15 % or less of the amino acids, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

The composition represents a novel retention time standard, called RePLiCal (Reversed-phase liquid chromatography calibrant) for monitoring reverse phase liquid chromatography. The peptides of SEQ ID NO.: 1 - 27 were specifically selected to cover a broad range of hydrophobicity such that they elute over a broader range than any available retention time standard, providing retention time standards and controls for particularly hydrophobic as well as hydrophilic peptides i.e. peptides eluting at the very end or the very beginning of the reverse-phase chromatography run. Thereby, they provide additional data points for standardization, which is of particular advantage for the iRT concept (Escher et al., 2012). Moreover, due to the high number of differently hydrophobic peptides, a greater density of reference points is provided throughout the run. This allows for high resolution monitoring and even realignment of chromatographic profiles in label-free liquid chromatography-mass spectrometry (LC-MS) based protein quantification studies. Moreover, as SEQ ID NO.: 1 - 27 provide reference peptides that sequentially elute from and are consistently dispersed throughout a reverse phase liquid chromatography gradient elution, they are particularly suitable for improving the implementation of dynamic adjustment of scheduled selected reaction monitoring (SRM) analysis. Additionally, as the peptides of SEQ ID NO.: 1 - 27 were selected for their particular stability, e.g by avoidance of methionine residues, tryptophan residues and cysteine residues as well as aspartic acid-proline, asparagine-proline and asparagine-glycine motifs, they show high reliability in their elution. For example, their elution sequence remains mostly unchanged when applying different chromatography parameters, e.g. different columns, solvent compositions or gradient profiles. This makes the retention time standard available for many different experimental setups and finally allows the comparison of results obtained by using different reverse-phase liquid chromatography parameters and/or instruments.

The term "gradient elution" as used herein refers to the process of extracting material from the chromatography column by changing the composition of the solvent (mobile phase) over time. The term "gradient profile" as used herein refers to the changes in the composition of the solvent. The term "gradient time" as used herein refers to the time during which the composition of the solvent is changed for extracting material from the chromatography column. In particular, this excludes the time for washing and equilibrating the chromatography column.

At least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof. By the presence of at least one peptide of each said group, regularly distributed reference points covering most of a reverse-phase chromatography run are provided. This is expected to be sufficient for standard applications.

Although SEQ ID NO.: 1 - 27 represent the most preferred R-peptides, variants of these peptides are likewise suitable for carrying out the invention, because a limited number of amino acid exchanges will not significantly alter a peptide's hydrophobicity and, thus, its elution profile compared to the original peptide. For example, short peptides of about 8 amino acids may comprise only one or two amino acid exchanges, whereas long peptides, due to the increased complexity of the peptide, may have more amino acid exchanges. Thus, a peptide of about 31 amino acids may comprise up to 9 amino acids exchanges. Preferably, the exchanges reflect the limitations considered when selecting the peptides of SEQ ID NO.: 1 - 27, e.g. methionine, tryptophan and cysteine residues should be excluded as well as aspartic acid-proline, asparagine-proline and asparagine-glycine motifs. In contrast, conservative mutations known to have minimal effect on retention time as e.g. glycine to alanine, glutamic acid to aspartic acid may be easily possible.

Disclosed are variants that differ from the original peptide in 25 % or less of the amino acids, preferably in 20 % or less of the amino acids, more preferred in 15 % or less of the amino acids.

Since the peptides of the invention cover a particularly broad range of hydrophobicity, they are suitable for liquid chromatography, in particular reverse-phase liquid chromatography, independently of the particular parameters used, e.g. composition of the stationary phase, composition of the solvent, column length, flow rate of the solvent or gradient profile. Therefore, depending on the parameters of the chromatography, use of a subset of peptides may be sufficient or even preferred. For example, for closely monitoring the resolution of a specific elution window, peptides may be selected covering that particular window. In contrast, when monitoring an entire chromatography gradient elution more generally, i.e. without the need of particular high resolution, a subset of e.g. 8 to 10 peptides, eluting at regular intervals during the entire chromatography gradient elution, may be preferred. These would allow recognition of general problems during the run without the need to monitor a high number of peptides. Finally, when using the chromatography to purify or analyse a particular peptide having a known retention time, R-peptides eluting about the same time or slightly before the peptide of interest, may be used to indicate the elution of the peptide. For experiments requiring a more stringent quality control and, thus, higher resolution monitoring, more than 8 peptides may be selected. Accordingly, in a preferred embodiment, the composition comprises at least 10, preferably at least 15, more preferred at least 20, most preferred 27 R-peptides.

In a preferred embodiment, each variant has a retention time that differs from the retention time of the original peptide by 10 % or less, preferably 5 % or less of the total gradient time. Amino acid exchanges for providing variants of SEQ ID NO.: 1 - 27, are preferably selected such that the retention time of the variant is not shifted by more than 10 % compared to the original peptide (within the same LC gradient elution) with respect to the total gradient time. For example, in case the total gradient time amounts to 40 minutes, the amino acid exchanges may result in a retention time shift of about ± 4 minutes or less of the variant in comparison to the original R-peptide, if applied in the same chromatography run. Depending on the aim of the chromatography, the stationary and/or the mobile phase may be specifically adopted, which will influence the retention time of any peptide including the R-peptides. Additionally, the retention times of peptides are influenced by the elution conditions, thereby allowing the optimization of chromatography resolution. For example, desorption of a peptide from the column may be achieved either using a stepwise or a linear continuous gradient profile, wherein the latter is preferred for a high resolution. For separating biomolecules from a complex sample, high resolution is usually required that may be achieved by using a rather shallow gradient (i.e. by increasing elution time). Thus, the total retention time of a given peptide may vary depending on the chromatographic conditions used. Despite that, however, the R-peptides were found to stably and reliably elute in their sequence even for various different chromatography parameters. Thus, a variant of any of SEQ ID NO.: 1 - 27 may show a slightly different retention time compared to the original R-peptide for the same chromatography conditions, but may still provide the advantages of the invention. This is in particular so, if the elution sequence of the R-peptides is maintained.

In a preferred embodiment, each R-peptide is selected from the group consisting of SEQ ID NO.: 1 or a variant thereof, SEQ ID NO.: 2 or a variant thereof, SEQ ID NO.: 3 or a variant thereof, SEQ ID NO.: 4 or a variant thereof, SEQ ID NO.: 5 or a variant thereof, SEQ ID NO.: 6 or a variant thereof, SEQ ID NO.: 7 or a variant thereof, SEQ ID NO.: 8 or a variant thereof, SEQ ID NO.: 9 or a variant thereof, SEQ ID NO.: 10 or a variant thereof, SEQ ID NO.: 11 or a variant thereof, SEQ ID NO.: 12 or a variant thereof, SEQ ID NO.: 13 or a variant thereof, SEQ ID NO.: 14 or a variant thereof, SEQ ID NO.: 15 or a variant thereof, SEQ ID NO.: 16 or a variant thereof, SEQ ID NO.: 17 or a variant thereof, SEQ ID NO.: 18 or a variant thereof, SEQ ID NO.: 19 or a variant thereof, SEQ ID NO.: 20 or a variant thereof, SEQ ID NO.: 21 or a variant thereof, SEQ ID NO.: 22 or a variant thereof, SEQ ID NO.: 23 or a variant thereof, SEQ ID NO.: 24 or a variant thereof, SEQ ID NO.: 25 or a variant thereof, SEQ ID NO.: 26 or a variant thereof, SEQ ID NO.: 27 or a variant thereof. Preferably, the composition comprises either the original R-peptide or a variant thereof, but not both.

In a preferred embodiment, at least one R-peptide is selected from each group comprising group 5a consisting of SEQ ID NO.: 12 - 15 and variants thereof, and group 5b consisting of SEQ ID NO.: 16 - 18 and variants thereof. This provides a more stringent monitoring of the chromatography run and thus its resolution.

In a preferred embodiment, at least one R-peptide is selected from group 7 consisting of SEQ ID NO.: 24 - 27. Peptides of SEQ ID NO.: 24 - 27 represent the most hydrophobic peptides of the composition. In a chromatography with a gradient profile of increasing concentrations of organic solvent, these R-peptides elute particularly late. By including the late eluting R-peptides, the quality of the gradient elution can be monitored until the elution of the peptide of interest or even further on. Therefore, they are preferably included into the composition, in case the chromatography run to be monitored is used to separate particularly hydrophobic peptides.

In a preferred embodiment, at least one R-peptide is selected from the group consisting of SEQ ID NO.: 1 and 2. Many LC instruments, in particular nano LC instruments, used in proteomic experiments are operated in a trap-elute configuration, whereby peptides are loaded onto a short trapping column having a large internal diameter and then eluted into a narrower and longer analytical column. The trapping column allows peptides to be loaded from the sample loop at higher flow rates than would be possible using the analytical column, helping to minimize band broadening and to aid in sample desalting. For this configuration to be effective, peptides must partition into the stationary phase so that they are not lost during the time that a column eluent is being directed to waste. In this regard the risk that very hydrophilic peptides will not bind effectively to the trapping column and thus will be lost in the column flow-through is particularly high. The inclusion of one or two very hydrophilic peptides allows the standard to be used for testing trapping column performance. Preferably, the composition comprises a peptide comprising SEQ ID NO.: 1 or a variant thereof and a peptide comprising SEQ ID NO.: 2 or a variant thereof, thus containing both peptides.

In a further aspect, disclosed is a polypeptide comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids. In particular, the invention relates to a polypeptide comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange. In this polypeptide, the peptides of the invention are concatenated using the QConCAT technology (Pratt et al., 2006) such that the peptides are combined into a single artificial protein. Within the polypeptide, the individual R-peptides may by concatenated in any sequence. For use as a retention time standard, the polypeptide is digested into the single R-peptides. In case the standard is used together with a proteomic sample, the polypeptide may be digested beforehand and the digest added to the sample, or the polypeptide may be added to the sample such that the sample and the polypeptide are digested together. As the R-peptides were specifically designed to terminate with a lysine residue, enzymatic digest by the two most common enzymes, i.e. endoprotease Lys-C and trypsin, is possible and yields the same collection of R-peptides. Moreover, the peptides were selected so as to produce peptide bonds that are predicted to be cleavable to minimize the probability of mis-cleavage. Finally, storage of the intact polypeptide significantly improves analysis as it avoids the disproportional loss of single peptides, e.g. due to undesirable adherence to surfaces because of high hydrophobicity.

In a preferred embodiment, the polypeptide comprises each R-peptide in a stoichiometry of 1:1. In case loss of protein occurs during storage, the peptides will still remain in the 1:1 stoichiometry prior to digestion such that all calibration points will be available even if the standard is stored for an extended time period.

In a preferred embodiment, the polypeptide further comprises a peptide located at the *N*-terminus, which comprises a methionine initiator residue. Thereby, the polypeptide may be prepared by heterologous expression in *E. coli.*

In a preferred embodiment, the polypeptide further comprises a peptide located at the C-terminus, which comprises a His-tag. This allows for high purity preparations of the polypeptide.

In a preferred embodiment, the polypeptide comprises SEQ ID NO.: 3 - 27, preferably further comprising SEQ ID NO.: 1 and/or SEQ ID NO.: 2.

In a further aspect, disclosed is a nucleic acid construct encoding for a polypeptide comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids. The construct is suitable to be introduced into a cell such that the cell expresses the polypeptide. To achieve this, the construct may be included into a plasmid, which is then introduced into a cell, e.g. by transformation. The cell then expresses the polypeptide, which can be purified from the cell's lysate.

In a further aspect, disclosed is a cell, preferably a bacterial cell, comprising a nucleic acid construct encoding for a polypeptide comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids. Such cells may be cultured in industrial scale to produce the polypeptide comprising the R-peptides.

In a further aspect, disclosed is a kit comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids. In particular, the invention relates to a kit comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange. Providing the peptides as a ready to use kit, either as single peptides or as a concatenated polypeptide, the retention time standard can be directly applied for research and analytical experiments.

In a further aspect, disclosed is the use of at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids, for reverse-phase liquid chromatography. In particular the invention relates to the use of at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, for reverse-phase liquid chromatography, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange. Reverse-phase liquid chromatography is the method of choice for separating and/or purifying biological molecules such as proteins and peptides. Accordingly, it has gained increasing importance in proteomic research and analysis. After absorption of the biomolecules to the hydrophobic stationary phase of the chromatographic column, selective desorption - depending on the hydrophobicity of the biomolecule - is achieved by gradient elution. The eluting molecules may be collected in narrow samples thereby separating the different biomolecules for further analysis. Alternatively, the eluate is directly subjected to analysis e.g. by mass spectrometry.

Adequate resolution and recovery is essential for successful reverse-phase liquid chromatography, both depending on the properties of the stationary and liquid phase including column selectivity, flow rate, gradient profile, column length and mobile phase composition. In summary, the parameters of the reverse-phase liquid chromatography define the point at which a given molecule desorbs from the column and elutes, thus the time a molecule takes to pass through the column (retention time). For testing the quality of a chromatography setup, quality control standards comprising peptides of known elution sequence and known relative elution times are used. The peptides of sequences SEQ ID NO.: 1 - 27 provide a high quality control standard as they cover a significantly broad hydrophobicity range and constant elution sequence for many chromatography conditions. In addition, the retention times of the R-peptides show an almost linear regression (Figure 5), which allows the transfer of assays between different liquid chromatography instruments and/or conditions, because it allows a direct comparison.

In a further aspect, disclosed is the use of at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids for "selected reaction monitoring" (SRM). In particular, the invention relates to the use of at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids for "selected reaction monitoring" (SRM), wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange. SRM, also referred to as "multiple reaction monitoring" (MRM), provides a targeted mass spectrometric approach using tandem quadrupole mass spectrometers (QqQ). For analysis of a specific target peptide, the first quadrupole mass analyzer is set to admit a single *m*/*z* value, namely that of the ionized target peptide (precursor) to the collision cell. In the collision cell, the precursor ion is fragmented by low energy collision-induced dissociation (CID) to generate specific product ions. The second quadrupole mass analyzer is then also fixed on one or more *m*/*z* value(s), namely the *m*/*z* value of the product ion(s), such that only the specific product ion(s) of interest that derive from the predefined precursor ion will have a stable trajectory to the detector. The two levels of *m*/*z* selection result in a high selectivity, a low background signal and a high duty cycle, providing the significant advantages of SRM. As a peptide to be detected needs to satisfy the *m*/*z* value of the first quadrupole and the generated product must correspond to *m*/*z value* of the second quadrupole, even co-eluting peptides may be distinguished, because they will be recognized by their different product ions (Holman et al., 2012). However, for an optimal analysis of a specific peptide of interest by SRM, certain parameters have to be known, namely the precursor ion-charge state to recognize a given peptide, the *m*/*z* value of the product ion generated by low energy CID of the precursor, the collision energy required to effect dissociation of the precursor and generate optimal signal intensity of the product, and the retention time of the peptide under the chromatographic conditions employed to facilitate scheduled acquisition. The retention time is particularly important for the so-called "scheduled SRM" (also referred to as scheduled or dynamic MRM, dMRM), wherein only the transitions for a specific peptide are detected in a time window around its expected elution from the chromatographic column. This provides the particular advantage of improved data quality and the ability to significantly increase the number of peptides targeted in a single analysis. Furthermore, the knowledge of a peptide's retention time increases the selectivity of the assay, because the signal can be differentiated as arising from the targeted peptide or the background based upon the peptides estimated elution time.

However, as the retention time of a single peptide is influenced by several parameters, monitoring the elution of the R-peptides allows a direct observation of the progress of chromatographic separation. Thereby the expected elution of a peptide of interest can be estimated. In particular, changes in the elution behaviour of the chromatographic column can be detected beforehand and, in case of irregularities, the expected elution time of a peptide of interest recalculated. This helps to avoid missing the elution of the peptide of interest, in particular to prevent elution of the peptide outside the time window scheduled for SRM analysis.

In a further aspect, disclosed is a method for monitoring reverse-phase chromatography, comprising the steps providing at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids, applying the R-peptides to a chromatography column, and determining the retention time of each peptide. In particular, the invention relates to a method for monitoring reverse-phase chromatography, comprising the steps providing at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, applying the R-peptides to a chromatography column, and determining the retention time of each peptide, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange.

The R-peptides may be provided as single peptides or as a concatenated polypeptide. Accordingly, they may be directly applied or upon digest of the concatenated polypeptide, respectively. Depending on the aim of the reverse-phase liquid chromatography, the R-peptides may be applied alone, for example for testing the behaviour of the chromatographic setup when developing a novel protocol, or they may be applied together with a sample for monitoring the chromatography run. For example, when used alone for testing a setup, the peptides may be applied in an amount of 5 to 100 fmol. When applied together with a sample, however, the amount of R-peptides may be chosen in respect to the total amount of protein contained in the sample. As the R-peptides were found to be readily detectable in a complex matrix even when representing only a small fraction of the total protein load, an addition of R-peptides in an amount corresponding to about 0.02 % of the total protein amount still led to reliable results. Nevertheless, different experiments may require different amounts of peptides, as for example non-targeted data dependent acquisition and data independent acquisition experiments are expected to require up to 0.5 % of the sample loaded on the column. Thus, the amount of R-peptides added to the sample may correspond to about 0.5 % to 0.02 % of the protein amount of the sample.

In case the peptides are provided as a concatenated protein and are supposed to be applied together with the sample to be analyzed, the concatenated protein may be previously digested or digested together with the sample. The latter is particularly preferred as the digest conditions will be identical for both the standard and the sample, such that the R-peptides and the target peptides are expected to be digested with the same efficiency. Finally, retention times may be determined either by using a UV detector or, if arranged in-line with a mass spectrometer, by measuring the mass spectrometry signal. In this respect, the method is particularly suitable for reverse-phase liquid chromatography in combination with scheduled SRM.

In a preferred embodiment, the chromatography is performed using a reverse-phase liquid chromatography instrument, preferably a HPLC instrument, more preferred a low pH reverse-phase liquid chromatography mass spectrometry (RP-LC-MS) instrument.

In a preferred embodiment, the reverse-phase chromatography is followed by selected reaction monitoring (SRM) mass spectrometry.

In a further aspect, disclosed is a method for estimating a retention time of a target peptide during a liquid chromatography run, comprising the steps providing at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids, adding the peptides to a sample comprising the target peptide, applying the sample to a chromatography column, determining the retention times of the R-peptides and estimating the retention time of the target peptide according to the determined retention times of the R-peptides. In particular, the invention relates to a method for estimating a retention time of a target peptide during a liquid chromatography run, comprising the steps providing at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 15 % or less of the amino acids, adding the peptides to a sample comprising the target peptide, applying the sample to a chromatography column, determining the retention times of the R-peptides and estimating the retention time of the target peptide according to the determined retention times of the R-peptides, wherein each variant does not comprise methionine, tryptophan, cysteine, an aspartic acid-proline motif, an asparagine-proline motif or an asparagine-glycine motif, and wherein each amino acid that differs from the original peptide is obtained by a conservative amino acid exchange. Retention times of peptides or proteins are influenced by various conditions of the chromatography. Thus, the actual retention time of a peptide of interest may divert from the expected retention time due to unintended changes of conditions, e.g. mobile or stationary phase composition, flow rate or gradient profile. Likewise, an adaption of the gradient profile may be recommended to increase resolution for an unexpected complex sample. Thus, monitoring of a chromatography run is advantageous as it helps to avoid an intended loss of material and, thus, data. For example, by monitoring the elution of R-peptides, an alteration in the elution profile of the chromatography will be recognized early enough to adapt the chromatography conditions, e.g. the shape or velocity of the gradient profile. Alternatively, the collection of samples or the time window for mass spectrometry analysis may be shifted, in case the peptide of interest will elute later or earlier than expected. This is particularly important for scheduled SRM wherein the mass spectrometric analysis is adapted to specific peptides of interest during limited time windows.

In a further aspect, disclosed is a method for estimating a retention time of a target peptide during liquid chromatography, comprising the steps providing a composition comprising at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids, adding a first part of the composition to a sample comprising the target peptide, applying the sample to a first chromatography setup, determining the retention times of the R-peptides and the target peptide on the first chromatography setup, applying a second part of the composition to a second chromatography setup, determining the retention times of the R-peptides on the second chromatography setup, calculating a regression line of the retention times of the R-peptides determined for the first and second chromatography setup, and estimating the retention time of the target peptide on the second liquid chromatography setup based on the regression line. The term "chromatography setup" refers to the combination of all instruments and parameters used for liquid chromatography. Thus, two chromatography setups may differ in the instruments used and/or the conditions of the run, e.g. in column composition or length, solvent composition or gradient profile. As the retention times of the peptides of the invention show an almost linear regression even under different chromatography conditions, they can be used for establishing a relationship between two chromatography setups in terms of peptide retention times. By means of this relationship, the retention time of a target peptide on a second chromatography setup can be estimated based on its retention time on a first chromatography setup. In detail, the elution profiles of the R-peptides on the first and the second chromatography setup are characterized. Then, the equation of the regression line relating the two elution profiles is established. The retention time of a target peptide on the second chromatography setup is finally estimated based on its retention time on the first chromatography setup using the equation of the regression. For example, the retention time of a set of peptides may be determined on a chromatography setup with a long discovery type gradient. To target these peptides in a SRM experiment, which usually employs shorter gradient profiles or even a different chromatography system, their expected retention times in the second setup could be calculated by the regression line relating the two elution profiles.

In a further aspect, disclosed is a method for correcting the *m*/*z* scale of high resolution accurate mass spectrometry data, comprising the steps providing at least 8 reference peptides (R-peptides) selected from the group consisting of SEQ ID NO.: 1 - 27 and variants thereof, wherein at least one R-peptide is selected from each group comprising group 2 consisting of SEQ ID NO.: 3 - 5 and variants thereof, group 3 consisting of SEQ ID NO.: 6 - 8 and variants thereof, group 4 consisting of SEQ ID NO.: 9 - 11 and variants thereof, group 5 consisting of SEQ ID NO.: 12 - 18 and variants thereof, and group 6 consisting of SEQ ID NO.: 19 - 23 and variants thereof, wherein each variant differs from the original peptide in 30 % or less of the amino acids, adding the R-peptides to a sample to be analyzed, analyzing the sample by liquid chromatography and mass spectrometry to obtain raw data on retention times of the R-peptides and the sample, and lock mass correcting the raw data using the *m*/*z* values of the R-peptides as reference points. Standardization points throughout the elution profile of the entire run can be used as lock masses to correct the *m*/*z* scale of high resolution accurate mass spectrometry data to improve total peptide identification in shotgun proteomics experiments. The R-peptides are spiked into the sample and analyzed together with the sample using a mass spectrometer (MS) instrument capable of acquiring high-resolution accurate mass data, as for example an orbitrap, time of flight (ToF) or ion cyclotron resonance (ICR) mass analyser, a quadrupole-time-of-flight (QTOF) analyser or a Fourier transform-ion cyclotron resonance (FT-ICR) analyser. The exact *m*/*z* value of the R-peptide is then used to lock mass correct the data in the truncated section of the elution profile of the entire run, following which the sections are recombined and searched against the same database as for the raw data. In summary, the use of the R-peptides essentially allows the acquired raw data to be corrected following mis-calibration, which is suitable for recognizing the drifting or complete loss of calibration during an analysis and, thus, is suitable to prevent data loss. Moreover, this also enables a more efficient use of instrument times as re-analysis is not required and prevents complete loss of data in sample limited situations. The addition of R-peptides in this situation is particularly advantageous for poorly characterized samples where knowledge of the expected endogenous peptides, which could be used for *m*/*z* scale recalibration, is not available.

In a further aspect, the invention relates to the use of a polypeptide according to the invention for determining the efficiency of digest by trypsin or Lys-C. As the polypeptide comprising the concatenated peptides does not have any peptide bonds that are predicted to be mis-cleaved and no mis-cleavage has been observed (at least for trypsin digestions), the polypeptide may be used for assessing the efficiency of protein digest. By adding the protein to the sample and digesting both together, the appearance of mis-cleaved R-peptides indicates an incomplete proteolysis. This can be of particular relevance as it may cause inaccurate results for quantification.

In a further aspect, the invention relates to the use of a polypeptide according to the invention for realigning data obtained by label-free protein quantification.

## Examples

### Material and Methods

*Peptide selection*

Candidate peptides were selected using in-house RP-nano uHPLC-nano ESI-$MS^E$ (reversed phase-nano-ultra-high performance liquid chromatography-mass spectrometry with elevated energy) data from tryptic digests of *Escherichia coli* and *Saccharomyces cerevisiae.* Peptides containing methionine, tryptophan, cysteine and *N*-terminal glutamine residues, and aspartic acid-proline, asparagine-proline and asparagine-glycine motifs were removed from the dataset. Arginine-terminating peptides were also excluded from consideration to ensure that the selected sequences would conform to both Lys-C and trypsin cleavage specificity once assembled into the QconCAT protein. Peptides with good elution profiles (minimal tailing, narrow peak width at FWHM and 10 % height) were chosen for further consideration. Candidates from throughout the gradient elution were selected. For peptides originating from *Saccharomyces cerevisiae,* conservative mutations were made to the amino acid sequences that have been reported to have minimal effect on retention times e.g. glycine to alanine, glutamic acid to aspartic acid. In addition, peptide with aspartic acid and glutamic acid in positions P4, P3, P2, P1', P2' and P3' (Schechter & Berger nomenclature) were permutated to move the acidic side chains further from the lysine residue and reduce the likelihood of missed cleavages in the final QconCAT protein. Candidate peptides were BLAST searched against the Non-redundant protein sequences (nr) database with prokaryotic organisms excluded to verify uniqueness as Lys-C or tryptic peptides, taking into consideration the inability of low-energy CID to differentiate leucine and isoleucine and also low resolving power instrumentation to discriminate between glutamine and lysine. Sequences that matched a Lys-C or tryptic peptide in any eukaryotic organism were discarded. The remaining 61 peptides were prepared by SPOT synthesis (JPT Peptide Technologies, Berlin, Germany) to enable evaluation of the novel sequences generated by conservative mutation and sequence permutation. Each peptide was resolubilised in 100 mM ammonium bicarbonate (AmBic): (MeCN) [80:20, v/v] according to the manufacturer's instructions to a final concentration of 1 nmol $\mu L^{-1}$. One $\mu$L was taken from each stock, pooled and dried to completeness using vacuum centrifugation. The peptides were then resolubilised using 100 mM AmBic:MeCN [80:20, v/v] to a final concentration of 1 pmol $\mu L^{-1}$, vortexed and sonicated for 5 min. A 1 in 10 dilution was performed using 0.1 % formic acid in $H_2O$:MeCN [97:3, v/v] and 1 $\mu$L analysed in triplicate by RP-nano uHPLC-$MS^E$ using a nanoACQUITY nano LC instrument coupled to a Synapt HDMS Q-ToF mass spectrometer (Waters Ltd., Elstree, UK) (full details can be found in the Supporting information). The data was evaluated and 27 consistently observed peptides eluting at regular time points throughout the gradient were selected for inclusion in the QconCAT protein.

*Recombinant expression of QconCAT protein and initial characterisation*

A gene encoding for RePLiCal was synthesised and cloned into the expression vector pET21a and provided as 3 $\mu$g of lyophilised powder (PolyQuant GmbH, Bad Abbach, Germany). The powder was solubilised in 30 $\mu$L of 10 mM Tris-HCl, pH 8.4. One microlitre of the solution was taken and the RePLiCal gene was transformed into *E. coli* JM109 (DE3) competent cells. A 25 $\mu$L aliquot of the *E. coli* cells was streaked onto a Luria agar (LA) with 50 $\mu g\ mL^{-1}$ ampicillin selection plate and incubated at 37 °C overnight as previously described. A single colony was picked and inoculated into 5 mL of Luria broth (LB) with 50 $\mu g\ mL^{-1}$ ampicillin and incubated at 37 °C overnight with shaking at 220 rpm. This culture was transferred to 500 mL of auto-induction media in a baffled flask (ForMedium, Hunstanton, UK) supplemented with 50 $\mu g\ mL^{-1}$ ampicillin and 2.8 $\mu moles\ mL^{-1}$ glucose and incubated at 30 °C for 20 h with shaking at 220 rpm. The cells were pelleted by centrifugation at 4500 rpm, 4°C for 15 min. The supernatant fraction was discarded and the cells resuspended in 2 mL of lysis buffer (25 mM Tris-HCl pH 8.0, 300 mM NaCl, 1 % Triton X-100, 10% glycerol and 1:1000 protease inhibitor cocktail). Cell lysis was performed on ice by sonication for six cycles of 30 s with 30 s cool down periods in between each cycle. A 1 mL portion of the total fraction was removed and stored on ice. The remaining 1mL of the total fractionation was centrifuged at 15,000 rpm, 4 °C for 15 min to pellet the lysed sample. The supernatant fraction containing the soluble proteins was removed and stored on ice. The cell pellet was resolubilised in 1 mL of lysis buffer and stored on ice. Twenty-five microlitre portions of the total (T), supernatant (S) and pellet (P) fractions were mixed 1:1 with 2x sodium dodecyl sulfate-polyacrylamide gel electrophoresis (SDS-PAGE) loading buffer (100 mM Tris-HCl pH 6.8, 4 % SDS [w/v], 20 % glycerol [w/v], 200 mM beta-mercaptoethanol and 0.2 % bromophenol blue [w/v]), loaded onto a 12 % gel and separated at a constant voltage of 160 V. The pellet fraction was purified using ammonium sulfate precipitation. Initially, cuts of 15, 30, 50 and 75 % ammonium sulfate were used. SDS-PAGE analysis as described above showed that the vast majority of the RePLiCal was contained in the 30 % cut. Further optimisation was performed using 20, 25 and 30 % cuts, which demonstrated that RePLiCal was most abundant in the 25 and 30 % cuts. Therefore, the final purification was performed with cuts of 20, 30 and 70 % ammonium sulfate. SDS-PAGE analysis was performed using 0.5 % of each sample, demonstrating that RePLiCal was present in the 30 % cut. To quantify RePLiCal in the purified preparation, SDS-PAGE was performed as described above using a bovine serum albumin (BSA) standard curve as a reference. Using the 1 in 4 dilution of purified RePLiCal, it was estimated that the final concentration was ~2 $\mu g\ \mu L^{-1}$, which is equivalent to -50 pmol $\mu L^{-1}$.

*Digestion of yeast lysate*

One hundred micrograms of whole Yeast (*S. cerevisiae*) cell lysate were solubilised in 0.1 % RapiGest SF, reduced with 3 mM dithiothreitol, alkylated with 9 mM iodoacetamide and digested with trypsin at an enzyme:substrate ratio of 1:50 [w/w] overnight at 37 °C. Addition of trifluoroacetic acid to 0.5 % [v/v] terminated the enzymatic reaction and degraded the RapiGest SF following incubation at 37 °C for 2 hr. The sample was centrifuged at 13,000 g/4 °C for 15 min and the cleared supernatant fraction removed for further analysis.

*RP-nano uHPLC-SRM-MS analysis of RePLiCal*

All experiments involving RePLiCal were performed using the experimental conditions detailed below unless stated otherwise in subsequent sections. Samples were diluted to the appropriate concentration for the given experiment in 0.1% formic acid in $H_2O$:MeCN [97:3, v/v] and analysed by RP-nano uHPLC-nano ESI-SRM-MS/MS on a nanoACQUITY nano LC instrument coupled to a Xevo TQ MS tandem quadrupole mass spectrometer (Waters Ltd., Elstree, UK). The samples were loaded onto a Symmetry C18 trapping column (5$\mu$m packing material, 180 $\mu$m x 20 mm) (Waters Ltd., Elstree UK). Trapping and desalting was performed for 3 min at a flow rate of 5 $\mu L\ min^{-1}$. The solvent conditions were 99.9 % A: 0.1% B (A = 0.1 % formic acid [v/v], B = 0.1 % formic acid in MeCN [v/v], except for the ion-pairing agent comparison experiment where acetic acid was used at 0.5 % [v/v]). Post-trapping and desalting, the peptides were eluted onto a HSS T3 nanoACQUITY C18 analytical column (1.8 $\mu$m packing material, 75 $\mu$m x 150 mm) (Waters Ltd., Elstree, UK) and separated using gradient elution. The gradient profile starting conditions were 97 % A: 3% B. The column was developed to 40 % B over either 10, 30, 60 or 90 min, then to 95 % B over 2 min, held at 95 % B for 2 min, reset to starting conditions over 1 min and then re-equilibrated over 15 min. The flow rate was constant at 300 nL $min^{-1}$ and the column oven temperature was 35 °C. The column effluent was introduced into a nano ESI source fitted with a PicoTip emitter (New Objective, Woburn, MA, USA). The polarity of the ionisation source was set to positive. The ionisation source was operated under the following conditions; capillary voltage, 3.0 kV; cone voltage, 30 V; source temperature, 70 °C, nanoflow gas, 0.1 bar; cone gas, 50 L $hr^{-1}$. SRM analysis was performed in scheduled mode with time windows of 1, 2, 3 and 4 min for the 10, 30, 60 and 90 min gradients respectively. Collision-induced dissociation (CID) was performed using argon as the collision gas. The collision energy to dissociate each peptide was calculated using a linear regression optimised for the tandem quadrupole instrument used.

*Transferability of RePLiCal across LC instrumentation*

Fifty femtomoles of RePLiCal was analysed by RP-nano uHPLC-nano ESI-MS/MS on a Ultimate 3000 RSLC nano LC instrument coupled to a QExactive quadrupole-orbital trap mass spectrometer (ThermoFisher Scientific, Hemel Hempstead, UK). The sample was loaded onto a PepMap100 C18 $\mu$-Precolumn (5$\mu$m packing material, 300 $\mu$m x 5 mm) (ThermoFisher Scientific, Hemel Hempstead, UK). Trapping and desalting was performed for 5 min at a flow rate of 5 $\mu L\ min^{-1}$ using 0.1 % trifluoroacetic acid in $H_2O$:MeCN [98:2, v/v] as the solvent. Post-trapping and desalting, the peptides were eluted onto an Easy-Spray C18 analytical column (2 $\mu$m packing material, 75 $\mu$m x 500 mm) (ThermoFisher Scientific, Hemel Hempstead, UK) and separated using gradient elution. The gradient profile starting conditions were 96.2 % A: 3.8 % B (A = 0.1 % formic acid [v/v], B = 0.1 % formic acid in 80:20 MeCN:$H_2O$ [v/v]). The column was developed to 50 % B over either 30 or 90 min, then to 99 % B over 2 min, held at 99 % B for 5 min, reset to starting conditions over 0.1 min and then re-equilibrated for 15 min. The flow rate was constant at 300 nL $min^{-1}$ and the column oven temperature was 35 °C. The column effluent was directed through the integrated nano ESI emitter with the ionisation source polarity set to positive. The ionisation source was operated under the following conditions; spray voltage, 2.0 kV; capillary temperature, 250 °C; sheath gas, 0 arbitrary units; auxiliary gas, 0 arbitrary units; sweep gas, 0 arbitrary units; S-lens RF level, 50 %. Data-dependent acquisition (DDA) was performed consisting of the acquisition of a full scan mass spectrum between *m*/*z* 300-2000 at a mass resolution of 70,000 FWHM at *m*/*z* 200. The automatic gain control was set to $1e^6$ ions with a maximum fill time of 250 ms. The top 10 most abundant peaks were selected for MS/MS using higher-energy collisional dissociation (HCD) with nitrogen as the collision gas. Product ion data was acquired at a mass resolution of 35,000 FWHM at *m*/*z* 200. The automatic gain control was set to $1e^5$ ions with a maximum fill time of 100 ms. The ion selection window was 2 *m*/*z* units and a normalised collision energy of 30 % was used. Dynamic exclusion was applied with a 20 s window.

*Recalibration of m/z scale using RePLiCal*

RePLiCal was spiked into a tryptic digest of a whole cell yeast lysate at ratio of 25 fmol:250 ng per μL. Four microlitres of sample was analysed by RP-nano uHPLC-nano ESI-MS/MS on a nanoACQUITY nano LC instrument (Waters Ltd., Elstree, UK) coupled to an Orbitrap XL linear ion trap-orbital trap mass spectrometer (ThermoFisher Scientific, Hemel Hempstead, UK). Trapping and separation of the peptides was performed as described for the RP-nano uHPLC-nano ESI-SRM-MS analyses described above except that the gradient time was 270 min. The column effluent was introduced into a nano ESI source fitted with a PicoTip emitter (New Objective, Woburn, MA, USA). The polarity of the ionisation source was set to positive. The ionisation source was operated under the following conditions; spray voltage, 2.8 kV; capillary voltage, 45 V; capillary temperature, 200 °C; sheath gas, 0 arbitrary units; auxiliary gas, 0 arbitrary units; sweep gas, 0 arbitrary units; tube lens, 140 V. Data-dependent acquisition (DDA) was performed consisting of the acquisition of a full scan mass spectrum between *m*/*z* 300-1700 at a mass resolution of 60,000 FWHM at *m*/*z* 400. The automatic gain control was set to $2e^5$ ions with a maximum fill time of 500 ms. The top 5 most abundant peaks were selected for MS/MS in the linear ion trap using CID with helium as the collision gas. The automatic gain control was set to $1e^5$ ions with a maximum fill time of 25 ms. The ion selection window was 3 *m*/*z* units, the activation Q was 0.250 and the activation time was 30 ms. A normalised collision energy of 35 % was used and WideBand activation was switched on. Dynamic exclusion was applied with a 60 s window. The data was miscalibrated and sliced into 27 sections, each containing a RePLiCal peptide, using the RecalOffline functionality in Xcalibur (ThermoFisher Scientific, Hemel Hempstead, UK). The data was recalibrated using the exact *m*/*z* value of the RePLiCal peptide and the resultant data converted to .mgf format using ProteoWizard. The .mgf files were combined, and along with a .mgf file created from the originally acquired data, searched against the *S*. *cerevisiae* strain S288C protein database downloaded from the *Saccharomyces* Genome Database (http://www.yeastgenome.org/download-data/sequence) using Mascot. The data was searched with a precursor ion mass tolerance of 10 ppm and a product ion mass tolerance of 0.6 Da. Carbamidomethylation of Cys was selected as a fixed modification and deamidation of Asn and Gln and oxidation of Met were chosen as variable modifications. Two missed cleavages were allowed. The false discovery rate (FDR) was set to 1 %.

## Results

*Design of RePLiCal*

The capability of the QconCAT methodology to enable the synthesis of artificial designer proteins allowed an optimised construct to be produced that, upon proteolysis, generated an ensemble of peptides suitable for the testing and standardisation of High-Performance Liquid Chromatography (HPLC) instrumentation used for bottom-up proteomics. This artificial protein was named RePLiCal. RePLiCal is a 379 amino acid residue, 39,193.3 Da protein constituting 27 peptides used for the calibration of HPLC instruments, an *N*-terminal sequence with a methionine initiator residue and a C-terminal His-tag for purification if high purity preparations are required (Figure 1). RePLiCal has been designed to contain only lysine-terminating peptides, such that the same complement of proteolytic fragments are generated upon enzymatic digestion using either Lys-C or trypsin; the two most commonly employed proteases in proteomics studies (Figure 2). Chemically reactive residues and motifs were avoided to aid stability of the protein during storage and sample preparation. Further, the sequences of the peptides were optimised such that no scissile bond was predicted to mis-cleave based on prediction by MC:pred. LC-$MS^E$ (Liquid Chromatography Mass Spectrometry with elevated Energy) supported this assertion as no missed cleaved peptides were identified from 100 fmol of RePLiCal tryptic digest. A single missed cleaved peptide, AAAPEPETETETSSKIVPEPQPK (SEQ ID NO.: 30), was detected from the same amount of Lys-C digested RePLiCal. The presence of a missed cleaved peptide in the Lys-C digest in not entirely unexpected despite prediction of cleavage efficiency using MC:pred as the algorithm was trained using tryptic peptides. Based on these observations, in addition to acting as a retention time (RT) calibrant, RePLiCal can also be used as a rudimentary standard for measuring efficiency of tryptic digests (and to a certain extent Lys-C digests by discounting the AAAPEPETETETSSKIVPEPQPK (SEQ ID NO.: 30), missed cleaved sequence): if missed cleaved RePLiCal peptides are observed, there is a high likelihood that proteolysis of more difficult to cleave peptide bonds in a given sample will be incomplete, which can lead to inaccuracy and imprecision in both labelled and label-free quantification studies. The ease of digestion also makes the use of RePLiCal favourable to the employment of standard proteins for benchmarking instrument performance, as the inter-batch variability in digestion will be diminished. Finally, the concatenation of the calibrant peptides into an artificial protein presents an ideal storage environment. It is known that peptides, particularly hydrophobic sequences, can adhere to surfaces over time, in some cases irreversibly. Therefore, RT standards stored as peptide mixtures can experience a loss of calibrations point due adherence of one or more of the analytes to a surface over time. As the peptides in RePLiCal are stored at the protein-level there is no opportunity for differential adsorption to take place: whilst loss of the protein can occur, the peptides will remain in a 1:1 stoichiometry prior to digestion, meaning that all calibration points will be available even when the standard is stored for an extended time period.

*Comparison of RePLiCal with commercially available peptide RT standards*

RePLiCal was directly compared to three commercially available peptide RT standards: iRT-Kit (Biognosys AG, Zurich, Switzerland), Peptide Retention Time Calibration Mixture (Pierce, Rockford, USA) and MS RT Calibration Mix (Sigma-Aldrich, Poole, UK). SRM assays were designed for each RePLiCal peptide by selecting the four most intense product ions with *m*/*z* values greater than 400 from LC-$MS^E$ data. Three or four transitions per peptide were monitored for the commercial RT standards as recommended by the manufacturer. Figure 3 shows the chromatograms for 10 fmol of each of the RT standards acquired under the same liquid chromatography (LC) conditions (columns, mobile phases, gradient profiles etc.). It is evident that the RePLiCal peptides elute over a wider range of RTs using the 30 min LC gradient profile than the peptides from the three commercially available kits: the first two peptides from RePLiCal elute before the initial peptide of any of the kits emerges from the column. Furthermore, RePLiCal provides between one and seven additional peptides that elute after the longest retained peptides in the commercially available kits. Additional data points for standardisation are therefore obtained during the earlier and latter parts of the gradient elution. This will be advantageous for the iRT concept (Escher et al., 2012), which so far cannot be used for peptides that have RTs before the first or after the last eluting reference peptide (Malmström et al., 2012). Therefore, the reference peptides encoded within RePLiCal will help extend the iRT concept by providing a greater number of calibration points over a wider range of elution times. RePLiCal also has a greater density of calibration points during the time span that is covered by each of the other RT kits, allowing enhanced characterisation of the LC gradient. This benefit, in concert with additional early and late eluting peptides, is likely to be advantageous in terms of instrument standardisation and realignment of data in label-free quantification studies. In addition, the high number of reference peptides provided by RePLiCal, which are interspersed consistently throughout the gradient, would allow more effective implementation of the dynamic adjustment of scheduled SRM analysis described by Gallien and co-workers (2012). This approach uses the RTs of the two most recently detected reference peptides to adjust the time windows for peptides due to elute later in the gradient. As RePLiCal provides more reference peptides, use of this standard should allow faster reaction to changes in RTs and thus more efficient correction of time scheduled SRM experiments. Further, the two earliest eluting peptides from RePLiCal, which emerge from the LC column before the first eluting peptide from any of the other RT kits, would allow better rescheduling of time windows early in the gradient when it is known that RTs are more variable.

*Comparison of empirical and predicted RTs of RePLiCal peptides*

Many algorithms to predict peptide RTs using low pH RP-HPLC on C18 columns have been described and demonstrated to be effective under certain experimental conditions. However, none of the models accounts for all phenomena resulting from the interaction of peptides with C18 stationary phases, such as the stabilisation of helical structures, and thus can be prone to error (Reimer et al., 2012). It was therefore hypothesised that prediction of the elution order and RTs of the RePLiCal peptides would differ significantly from that observed experimentally. The BioLCCC model was chosen for comparison due to the ability of the *in silico* predictor to be programmed to match the experimental conditions under which RePLiCal was analysed. Figure 4 shows that correlation between predicted and experimental RTs is reasonably strong. However, for accurate prediction of unknown RTs, the equation for the regression line should be y = x. This was not observed and poor prediction of RTs was achieved using the BioLCCC model, with an average of a 1.81 min error in the predicted RT. The scatter of data points around the regression line also demonstrates that prediction of elution order is incorrect, thus suggesting that selectivity of separation is also poorly predicted. The observed discrepancies between the predicted and empirical data emphasises the need for reliable standards to assess LC conditions and performance experimentally.

*Transferability of RePLiCal across gradient profiles and LC instrumentation*

Figure 5 shows the retention times of the 27 RePLiCal peptides on four different length gradients (10, 30, 60 and 90 min gradient time, all 3-40% 0.1% formic acid in MeCN). Using the 30 min gradient as the reference, it was demonstrated that there was a high degree of proportionality in the transfer of RTs from the reference gradient to the other three that were performed due to the observation of a linear relationship in measured retention times. The linear relationship in RTs for the RePLiCal peptides for each individual gradient shows that the peptides are suited to characterising the gradient, which itself was linear. The observance of good linearity means that it should be possible to predict RTs of peptides on a new gradient by knowing their RT relative to the RePLiCal peptides on a reference gradient. Then, by establishing the linear relationship between the reference and new gradient by a single analysis of RePLiCal, large scale transfer of RTs of other peptides can be performed. This is particularly advantageous for adjusting scheduled SRM assays for different LC conditions on the same instrument (*vide infra*), or transporting the assays between different LC instruments (Holman et al., 2012). The ability of RePLiCal to allow the transfer of assays between different LC instruments was confirmed through analysis on platforms from two different manufacturers (Figure 6). Again, good linearity was observed, although slight changes in selectivity were evident, particularly during the later part of the gradient. However, the changes in RT due to differential selectivity are minor and do not cause a significant deviation from perfect linearity, suggesting a negligible effect on transferring RTs from one instrument to another would be observed. Observation of differentially selectivity, as evinced by changes in RT, was also observed when RePLiCal was analysed on a range of LC gradients (Figure 5). For the pair of peptides, AVTTLAEAVVAATLGPK (SEQ ID NO.: 24) and IAFFESSFLSYLK (SEQ ID NO.: 25), a change in elution order was observed for the 60 min and 90 min gradients compared to the 10 and 30 min gradients. Likewise, the elution order for TQLIDVEIAK (SEQ ID NO.: 14) and LTVLESLSK (SEQ ID NO.: 15) is switched on the 90 min gradient compared to the other three employed. Rationalisation of this observation can be provided by the linear solvent strength (LSS) theory (Equation 1) (Spicer et al., 2010).

**Equation 1** $$\log k = \log k_0 - S\phi$$

*k* is the retention factor at a given organic solvent volume fraction $\phi$ (in this study, % MeCN/100) in the mobile phase and $k_0$ is the retention factor with a pure aqueous mobile phase. The *S* parameter is a constant for a particular peptide at a given $\phi$ value and essentially measures the rate of change of the retention factor as a function of changing organic solvent composition of the mobile phase (Glajch et al., 1986). This model has therefore been used to explain differences in selectivity for peptide separations as a function of gradient slope *i.e.* rate of change of organic solvent, with peptides whose S curves intersect showing reversals in retentivity either side of a critical concentration of organic solvent. The rate at which this critical concentration is reached *i.e.* the slope of the gradient, defines which of the two peptides is the first to elute from the column (Spicer et al., 2010). Therefore, it can be concluded that the peptides AVTTLAEAVVAATLGPK (SEQ ID NO.: 24) and IAFFESSFLSYLK (SEQ ID NO.: 25), and TQLIDVEIAK (SEQ ID NO.: 14), and LTVLESLSK (SEQ ID NO.: 15), have S curves that intersect and thus demonstrate differential selectivity as a function of gradient slope. Practically, this is unlikely to be a problem as the observed deviation from the linear relationship between different gradients was minor. This suggests that good prediction of retention times on different gradients is eminently possible even when the RePLiCal peptides demonstrating differential selectivity are employed as reference points.

*Testing of trapping column performance using RePLiCal*

Many nano LC instruments used in proteomics experiments are operated in a trap-elute configuration, whereby peptides are loaded onto a short, larger internal diameter trapping column and then eluted onto a narrower, longer analytical column. The trapping column allows peptides to be loaded from the sample loop at higher flow rates than would be possible using the analytical column, helping to minimise band broadening. It also acts as a desalting column by loading the sample in high aqueous containing mobile phase, which will cause selective partitioning of peptides into the stationary phase whilst salt will be unretained, and directing the column effluent to waste for a period of time. For this configuration to be effective, peptides must partition into the stationary phase so that they are not lost during the time that the column effluent is being directed to waste. In particular, there is a risk that very hydrophilic peptides will not bind effectively to the trapping column and will be lost in the column flow-through. The inclusion of two very hydrophilic peptides in RePLiCal, VTASGDDSPSGK (SEQ ID NO.: 1) and ALAEDEGAK (SEQ ID NO.: 2), can allow the standard to be used to test trapping column performance. Figure 7 shows the signal intensities of these two peptides relative to the next eluting peptide, SSYVGDEASSK (SEQ ID NO.: 4), on both a faulty trapping column and one that is functioning optimally. It is evident that using the faulty column, the signal intensities for VTASGDDSPSGK (SEQ ID NO.: 1) and ALAEDEGAK (SEQ ID NO.: 2) are significantly decreased relative to SSYVGDEASSK (SEQ ID NO.: 4) (both <1 % relative intensity) than when the trapping column is effectively trapping hydrophilic peptides, which results in relative intensities of approximately 10 % and 40 % for VTASGDDSPSGK (SEQ ID NO.: 1) and ALAEDEGAK (SEQ ID NO.: 2) respectively compared to SSYVGDEASSK (SEQ ID NO.: 4). Therefore, the first two eluting peptides in RePLiCal can be used as rapid indicators of poor trapping column performance when their signal intensities decrease significantly relative to SSYVGDEASSK (SEQ ID NO.: 4) and thus provide feedback as to whether replacement is required.

*Detection of RePLiCal in a complex matrix*

To test the utility in proteomics experiments, RePLiCal peptides were spiked into a tryptic digest of a whole cell yeast lysate and analysed by nLC-nESI-SRM-MS. Figure 8 shows that all 27 RePLiCal peptides in 5 fmol quantities were detectable with good signal-to-background ratio (≥ 29:1) in a background of 1 μg of yeast tryptic peptides. This demonstrates that RePLiCal is readily detectable in a complex matrix and can be added to the sample in minimal amounts (5 fmol is equivalent to approximately 200 pg and represents a 0.02% increase in protein load on column), thus itself not increasing the complexity of the sample significantly. A greater quantity of RePLiCal would be required to ensure detection in non-targeted data-dependent acquisition (DDA) and data-independent acquisitions (DIA) experiments, but sufficient amounts are still unlikely to increase the complexity of the sample loaded on column by greater than 0.5%.

*Repeatability of RePLiCal performance over extended analysis times*

To assess the repeatability of RePLiCal performance, sixty consecutive injections of 50 fmol RePLiCal in 1 μg of yeast tryptic peptides were loaded onto the LC column and analysed using a 30 min LC gradient (> 2 days instrument time with wash and re-equilibration steps). Table 1 shows that the relative standard deviations (RSDs) of the RTs were below 1.2% for all peptides, demonstrating that even over prolonged analysis times high repeatability in RT measurement is obtained. Further, all but two of the peptides showed less than a 16% RSD in the peak width (FWHM), showing that the vast majority of the standards gave very consistent peak shapes. The two peptides that gave values exceeding 20%, TSAESILTTGPVVPVIVVK (SEQ ID NO.: 20) and IAFFESSFLSYLK (SEQ ID NO.: 25), were longer retained species, thus giving more scope for band broadening, but nonetheless still gave good peak shapes with the variation in their widths in absolute terms being of the order of a few seconds. Therefore, the good levels of repeatability observed provide confidence that RePLiCal can be used to monitor LC performance longitudinally and that deviation from precise measurements in terms of RTs and peak widths will give rapid feedback on problems with LC instrumentation.

| SEQ ID NO.: | Peptide sequence | Average RT / min | RSD RT / min | Average Peak width (FWHM) / s | RSD Peak width (FWHM) / s |
|---|---|---|---|---|---|
| 1 | VTASGDDSPSGK | 12.55 | 1.14 | 5.17 | 3.00 |
| 2 | ALAEDEGAK | 13.75 | 0.93 | 5.41 | 8.98 |
| 3 | ASADLQPDSQK | 14.65 | 0.83 | 8.00 | 1.96 |
| 4 | SSYVGDEASSK | 14.67 | 0.82 | 7.72 | 2.63 |
| 5 | AAAPEPETETETSSK | 14.97 | 0.80 | 7.61 | 1.82 |
| 6 | IVPEPQPK | 15.89 | 0.71 | 6.13 | 13.03 |
| 7 | GAIETEPAVK | 16.90 | 0.69 | 7.11 | 3.63 |
| 8 | FHPGTDEGDYQVK | 17.60 | 0.66 | 6.35 | 5.51 |
| 9 | VGYDLPGK | 19.08 | 0.59 | 5.50 | 15.10 |
| 10 | SAGGAFGPELSK | 20.22 | 0.57 | 9.76 | 11.39 |
| 11 | TASEFESAIDAQK | 20.88 | 0.53 | 5.22 | 10.20 |
| 12 | GVNDNEEGFFSAK | 22.42 | 0.51 | 6.25 | 15.30 |
| 13 | VGLFAGAGVGK | 23.12 | 0.49 | 7.39 | 4.82 |
| 14 | TQLIDVEIAK | 23.90 | 0.44 | 6.34 | 1.51 |
| 15 | LTVLESLSK | 24.17 | 0.45 | 8.19 | 4.02 |
| 16 | LAPDLIVVAQTGGK | 25.10 | 0.40 | 6.20 | 12.61 |

(continued)

| SEQ ID NO.: | Peptide sequence | Average RT / min | RSD RT / min | Average Peak width (FWHM) / s | RSD Peak width (FWHM) / s |
|---|---|---|---|---|---|
| 17 | LTIAPALLK | 25.64 | 0.41 | 7.44 | 8.68 |
| 18 | ILTDIVGPEAPLVK | 26.51 | 0.39 | 6.25 | 6.18 |
| 19 | LTIEEFLK | 28.56 | 0.47 | 7.26 | 8.55 |
| 20 | TSAESILTTGPVVPVIVVK | 29.62 | 0.29 | 6.92 | 23.49 |
| 21 | ISSIDLSVLDSPLIPSATTGTSK | 30.55 | 0.31 | 7.61 | 2.76 |
| 22 | AGLEFGTTPEQPEETPLDDLAETDFQTFSGK | 31.72 | 0.32 | 8.34 | 5.27 |
| 23 | VVSLPDFFTFSK | 32.23 | 0.39 | 8.10 | 5.61 |
| 24 | AVTTLAEAVVAATLGPK | 33.34 | 0.49 | 8.53 | 13.53 |
| 25 | IAFFESSFLSYLK | 34.13 | 0.55 | 8.66 | 24.25 |
| 26 | SSIPVFGVDALPEALALVK | 34.82 | 0.30 | 6.37 | 12.74 |
| 27 | FLSSPFAVAEVFTGIVGK | 36.66 | 0.42 | 8.72 | 5.63 |

*Recalibration of m/z scale using RePLiCal*

The characteristic of RePLiCal to provide standardisation points throughout the LC gradient prompted an investigation as to whether the peptides can be used as lock masses to correct the *m/z* scale of high-resolution accurate mass spectrometry (MS) data. This approach has been previously demonstrated to improve total protein identifications in shotgun proteomics experiments (Mirzaei et al., 2009). RePLiCal was spiked into a whole cell yeast lysate and analysed over a 4.5 hour LC gradient using a calibrated linear ion trap-orbital trap mass spectrometer. The raw data was searched and enabled the identification of 6410 peptides at a 1% FDR, which were attributed to a total of 926 proteins. The raw data was then miscalibrated by 0.125 *m/z* using the middle eluting peptide from RePLiCal, TQLIDVEIAK (SEQ ID NO.: 14), as the reference point. Following this, the miscalibrated data was split into several sections, each containing a single RePLiCal peptide at the mid-point of the section in the time dimension. The exact *m/z* of the RePLiCal peptide was used to lock mass correct the data in the truncated section of the LC gradient, following which the sections were recombined and searched using the same database search parameters as for the raw data. At a 1% FDR, 6374 peptides were identified, leading to the identification of 922 proteins. Whilst an improvement in protein identifications was not observed as described by Mirzaei and co-workers (this may be a function of the quality of the initial instrument *m/z* scale calibration), the use of RePLiCal essentially allowed the rebuilding of the acquired raw data following miscalibration. This orchestrated scenario replicates the drifting or complete loss of calibration during an analysis, and demonstrates the utility of RePLiCal to prevent data loss. This can enable more efficient use of instrument time as reanalysis is not required, and prevents complete loss of data in sample-limited situations. The addition of a standard such as RePLiCal in this situation is particularly advantageous for poorly characterised samples where knowledge of the expected endogeneous peptides, which could be used for *m/z* scale recalibration, is not available.

*Comparison of formic acid and acetic acids as ion-pairing agents*

Typically, low pH RP-LC-MS is performed using formic acid as the ion-pairing agent due to its volatility, and hence compatibility with the ESI process. However, acetic acid may be used as an alternative. RePLiCal was analysed on 10, 30, 60 and 90 min gradients using 0.1% formic acid and 0.5% acetic acid to compare performance using the different ion-pairing agents. Identical elution orders were seen with both acids, indicating that no ion-pairing agent changes in selectivity occurred (Figure 9). Comparison of the peak widths (FWHM) showed no statistically significant differences at the 1% confidence level using the Mann-Whitney U test. These observations demonstrate that the performance of RePLiCal is equivalent under both LC conditions, and therefore can be used as a standard irrespective of whether formic acid or acetic acid is used as the ion-pairing agent.

*Prediction of RTs using RePLiCal*

The use of RePLiCal allows the establishment of the relationship between two LC gradients in terms of peptide elution times (Figure 5). It was hypothesised that using these relationships, the RT of a target peptide could be predicted by knowing its RT on one gradient characterised by RePLiCal, and then using the equation of the regression line relating this gradient to another (as determined using RePLiCal) to calculate its expected RT. In a practical sense, a relevant scenario would be the measurement of the retention time of a set of peptides on a long discovery-type LC gradient. If these peptides were then to be targeted in a SRM experiment, which typically employ shorter LC gradients and often require a different LC-MS system, determination of their RTs for scheduling would be required. This can be time-consuming, particularly when methods for large numbers of peptides are being developed. To demonstrate the utility of RePLiCal in predicting RTs on different gradients, the elution times of RePLiCal and 100 peptides from moderate-to-high abundance proteins in yeast targeted using transitions selected from SRMAtlas were determined on 30 min LC gradient. Subsequently, the RTs of the RePLiCal peptides were recorded on a 60 min LC gradient and a linear regression linking the two gradients calculated. Using this linear regression, the RTs of the 100 yeast peptides were predicted and scheduled SRM methods using time windows of 1.5, 2, 2.5 and 3 min created. Each method was run in hexaplicate with detection of a peptide being regarded as the full elution profile of the peak being within the time window in all six analyses. Using a very narrow 1.5 min time window, this criterion was only satisfied for 67% of the peptides. However, lengthening the time window to 2 min led to successful detection of 97% of the peptides. Further widening of the time windows to 2.5 min and 3 min allowed successful detection of 99% and 100% of the peptides respectively. This shows that characterisation of two different LC gradients using RePLiCal allows successful prediction and scheduling of RT windows for a large number of peptides whose RTs have been determined using a different LC gradient and/or instrumentation, thus allow efficient transfer between discovery and targeted proteomics experiments.

RePLiCal variants

Plasmid DNA constructs (expression plasmid pET21a) each encoding for a polypeptide comprising the RePLiCal peptides (R-peptides) in a different sequence were generated and transformed into *E.coli* strain BL21(D3).

The DNA construct of SEQ ID NO.: 31 encoded for the polypeptide of SEQ ID NO.: 32 comprising the R-peptides in the sequence SEQ ID NO.: 1, 2, 3, 4, 5, 6, 7, 8, 9, 10, 11, 12, 13, 14, 15, 16, 17, 18, 19, 20, 21, 22, 23, 24, 25, 26, 27.

The first variant of the polypeptide (SEQ ID NO.: 34) was encoded by the DNA construct of SEQ ID NO.: 33 and comprised the R-peptides in the following sequence: SEQ ID NO.: 7, 21, 18, 8, 10, 17, 25, 19, 20, 15, 12, 26, 14, 2, 23, 24, 4, 3, 1, 11, 22, 16, 6, 5, 13, 27, 9.

The second variant of the polypeptide (SEQ ID NO.: 36) was encoded by the DNA construct of SEQ ID NO.: 35 and comprised the R-peptides in the following sequence: SEQ ID NO.: 19, 27, 5, 18, 7, 6, 16, 2, 11, 12,17, 22, 10, 4, 3, 15, 24, 8, 9, 21, 23, 20, 25, 26, 14, 1, 13.

For each variant, six different cultures of transformed *E.coli* were grown in 1 ml LB-medium, supplemented with 50 $\mu$g/ml ampicillin (37°C, 180 rpm). Protein expression was induced at OD600 = 0.6 by addition of 1 mM Isopropyl-$\beta$-D-thiogalactopyranosid (IPTG). After induction, cultures are incubated for 3 h and expression levels of RePLiCal polypeptides tested by SDS-PAGE and Coomassie Blue staining. For each RePLiCal variant the best *E.coli* clone was used for further characterization by western blotting, wherein the first variant showed the strongest and most specific expression.

Therefore, the first variant was further investigated for solubility of expression. Cell pellets of 0.5 L culture were suspended in 1 ml cell lysis buffer and cells lysed by sonication. The supernatant and pellet of the cell lysate were analysed by SDS-PAGE, showing that the first variant is partially expressed in soluble form at 37°C in 0.5 L LBAmp-culture.

The first variant was then expressed in *E.coli* in BL21(DE3) and purified by ion metal affinity chromatography (IMAC). Cells were grown in 2 L medium at 37°C until $OD_{600}$ reached 0.6. Protein expression wass induced by addition of 1 mM IPTG and expression continued for 3.5 h at 37°C. Cells were harvested by centrifugation, disrupted by sonification and resulting cell lysate purified by Ni-Sepharose ($Ni_{nat}$). The insoluble material was suspended in 4 ml binding buffer (50 mM NaP, pH 7.4, 500 ml NaCl, 40 mM Imidazole, 1 mM dithiothreitol (DTT)) and samples kept on ice for 2 h to ensure proper solubilization of inclusion bodies. After centrifugation (17000 x g, 10 min, 4°C) the supernatant was loaded onto the equilibrated resin ($Ni_{den}$). The column was washed 2 times (Wash 1 = 4 ml, Wash 2 = 8 ml) with binding buffer to remove unbound material. Target proteins were eluted with elution buffer (50 mM NaP, pH 7.4, 500mM Imidazole, 2 mM DTT) in 6 fractions of 1 ml. Samples for SDS-PAGE were dialysed against 0.1% (v/v) trifluoroacetic acid (TFA).

To investigate the solubility of the purified protein in the sample dialysis buffer (0.1% (v/v) TFA, 1mM DTT), 100$\mu$l of the elution fractions 3 and 4 from $Ni_{den}$ were centrifuged after dialysis. The pellet and the supernatant were analysed separately, showing that nearly the complete protein remains soluble after dialysis.

Western blotting experiments detecting Poly-His-Tag showed a higher purity of the preparation from inclusion bodies compared to the purification from soluble fraction of cell lysate. Additionally, no degradation products were detected, indicating that the dialysis buffer (0.1% (v/v) TFA, 1mM DTT) is likewise suitable for storage.

**References**

Burkhart, J. M.; Premsler, T.; Sickmann, A., Quality control of nano-LC-MS systems using stable isotope-coded peptides. Proteomics 2011, 11, 1049-1057.

Escher, C.; Reiter, L.; MacLean, B.; Ossola, R.; Herzog, F.; Chilton, J.; MacCoss, M. J.; Rinner, O., Using iRT, a normalized retention time for more targeted measurement of peptides. Proteomics 2012, 12, 1111-1121.

Gallien, S.; Peterman, S.; Kiyonami, R.; Souady, J.; Duriez, E.; Schoen, A.; Domon, B., Highly multiplexed targeted proteomics using precise control of peptide retention time. Proteomics 2012, 12, 1122-1133.

Glajch, J. L.; Quarry, M. A.; Vasta, J. F.; Snyder, L. R., Separation of peptide mixtures by reversed-phase gradient elution. Use of flow rate changes for controlling band spacing and improving resolution. Anal. Chem. 1986, 58, 280-285.

Holman, S. W.; Sims, P. F. G.; Eyers, C. E., The use of selected reaction monitoring in quantitative proteomics. Bioanalysis 2012, 4, 1763-1786.

Krokhin, O. V., Sequence-specific retention calculator. Algorithm for peptide retention prediction in ion-pair RP-HPLC: Application to 300- and 100-Å pore size C18 sorbents. Anal. Chem. 2006, 78, 7785-7795.

Krokhin, O. V.; Spicer, V., Peptide retention standards and hydrophobicity indexes in reversed-phase high-performance liquid chromatography of peptides. Anal. Chem. 2009, 81, 9522-9530.

Malmström, L.; Malmström, J.; Selevsek, N.; Rosenberger, G.; Aebersold, R., Automated workflow for large-scale selected reaction monitoring experiments. J. Proteome Res. 2012, 11, 1644-1653.

Mirzaei, H.; Brusniak, M.-Y.; Mueller, L. N.; Letarte, S.; Watts, J. D.; Aebersold, R., Halogenated peptides as internal standards (H-PINS): Introduction of an MS-based internal standard set for liquid chromatography-mass spectrometry. Mol. Cell. Proteomics 2009, 8, 1934-1946.

Moruz, L.; Staes, A.; Foster, J. M.; Hatzou, M.; Timmerman, E.; Martens, L.; Käll, L., Chromatographic retention time prediction for posttranslationally modified peptides. Proteomics 2012, 12, 1151-1159.

Pratt, J. M.; Simpson, D. M.; Doherty, M. K.; Rivers, J.; Gaskell, S. J.; Beynon, R. J., Multiplexed absolute quantification for proteomics using concatenated signature peptides encoded by QconCAT genes. Nat. Protoc. 2006, 1, 1029-1043.

Reimer, J.; Spicer, V.; Krokhin, O. V., Application of modern reversed-phase peptide retention prediction algorithms to the Houghten and DeGraw dataset: Peptide helicity and its effect on prediction accuracy. J. Chromatogr. A. 2012, 1256, 160-168.

Spicer, V.; Grigoryan, M.; Gotfrid, A.; Standing, K. G.; Krokhin, O. V., Predicting retention time shifts associated with variation of the gradient slope in peptide RP-HPLC. Anal. Chem. 2010, 82, 9678-9685.

## Claims

**1.** A composition comprising at least 8 reference peptides (R-peptides) selected from the group consisting of SEQ ID NO.: 1 - 27 and variants thereof, wherein at least one R-peptide is selected from each group comprising

- group 2 consisting of SEQ ID NO.: 3 - 5 and variants thereof,
- group 3 consisting of SEQ ID NO.: 6 - 8 and variants thereof,
- group 4 consisting of SEQ ID NO.: 9 - 11 and variants thereof,
- group 5 consisting of SEQ ID NO.: 12 - 18 and variants thereof, and

- group 6 consisting of SEQ ID NO.: 19 - 23 and variants thereof,
wherein each variant differs from the original peptide in 15 % or less of the amino acids,
wherein each variant does not comprise methionine, tryptophan, cysteine, an aspartic acid-proline motif, an asparagine-proline motif or an asparagine-glycine motif, and
wherein each amino acid that differs from the original peptide is obtained by a conservative amino acid exchange.

**2.** The composition of claim 1, wherein the composition comprises at least 10, preferably at least 15, more preferred at least 20, most preferred 27 R-peptides.

**3.** The composition of claim 1 or claim 2, wherein at least one R-peptide is selected from group 7 consisting of SEQ ID NO.: 24 - 27.

**4.** The composition of any of claims 1 to 3, wherein at least one R-peptide is selected from group 1 consisting of SEQ ID NO.: 1 and 2.

**5.** A polypeptide comprising at least 8 reference peptides (R-peptides) selected from the group consisting of SEQ ID NO.: 1 - 27 and variants thereof, wherein at least one R-peptide is selected from each group comprising

- group 2 consisting of SEQ ID NO.: 3 - 5 and variants thereof,
- group 3 consisting of SEQ ID NO.: 6 - 8 and variants thereof,
- group 4 consisting of SEQ ID NO.: 9 - 11 and variants thereof,
- group 5 consisting of SEQ ID NO.: 12 -18 and variants thereof, and
- group 6 consisting of SEQ ID NO.: 19 - 23 and variants thereof,
wherein each variant differs from the original peptide in 15 % or less of the amino acids,
wherein each variant does not comprise methionine, tryptophan, cysteine, an aspartic acid-proline motif, an asparagine-proline motif or an asparagine-glycine motif, and
wherein each amino acid that differs from the original peptide is obtained by a conservative amino acid exchange.

**6.** The polypeptide of claim 5, wherein the polypeptide comprises each R-peptide in a stoichiometry of 1:1.

**7.** A kit comprising at least 8 reference peptides (R-peptides) selected from the group consisting of SEQ ID NO.: 1 - 27 and variants thereof, wherein at least one R-peptide is selected from each group comprising

- group 2 consisting of SEQ ID NO.: 3 - 5 and variants thereof,
- group 3 consisting of SEQ ID NO.: 6 - 8 and variants thereof,
- group 4 consisting of SEQ ID NO.: 9 - 11 and variants thereof,
- group 5 consisting of SEQ ID NO.: 12 - 18 and variants thereof, and
- group 6 consisting of SEQ ID NO.: 19 - 23 and variants thereof,
wherein each variant differs from the original peptide in 15 % or less of the amino acids,
wherein each variant does not comprise methionine, tryptophan, cysteine, an aspartic acid-proline motif, an asparagine-proline motif or an asparagine-glycine motif, and
wherein each amino acid that differs from the original peptide is obtained by a conservative amino acid exchange.

**8.** Use of at least 8 reference peptides (R-peptides) selected from the group consisting of SEQ ID NO.: 1 - 27 and variants thereof, wherein at least one R-peptide is selected from each group comprising

- group 2 consisting of SEQ ID NO.: 3 - 5 and variants thereof,
- group 3 consisting of SEQ ID NO.: 6 - 8 and variants thereof,
- group 4 consisting of SEQ ID NO.: 9 - 11 and variants thereof,
- group 5 consisting of SEQ ID NO.: 12 - 18 and variants thereof, and
- group 6 consisting of SEQ ID NO.: 19 - 23 and variants thereof,
wherein each variant differs from the original peptide in 15 % or less of the amino acids, for reverse-phase liquid chromatography,
wherein each variant does not comprise methionine, tryptophan, cysteine, an aspartic acid-proline motif, an asparagine-proline motif or an asparagine-glycine motif, and
wherein each amino acid that differs from the original peptide is obtained by a conservative amino acid exchange.

**9.** Use of at least 8 reference peptides (R-peptides) selected from the group consisting of SEQ ID NO.: 1 - 27 and variants thereof, wherein at least one R-peptide is selected from each group comprising

- group 2 consisting of SEQ ID NO.: 3 - 5 and variants thereof,
- group 3 consisting of SEQ ID NO.: 6 - 8 and variants thereof,
- group 4 consisting of SEQ ID NO.: 9 - 11 and variants thereof,
- group 5 consisting of SEQ ID NO.: 12 - 18 and variants thereof, and
- group 6 consisting of SEQ ID NO.: 19 - 23 and variants thereof,

wherein each variant differs from the original peptide in 15 % or less of the amino acids, for selected reaction monitoring (SRM),
wherein each variant does not comprise methionine, tryptophan, cysteine, an aspartic acid-proline motif, an asparagine-proline motif or an asparagine-glycine motif, and
wherein each amino acid that differs from the original peptide is obtained by a conservative amino acid exchange.

**10.** A method for monitoring reverse-phase chromatography, comprising the steps

- providing at least 8 reference peptides (R-peptides) selected from the group consisting of SEQ ID NO.: 1 - 27 and variants thereof, wherein at least one R-peptide is selected from each group comprising

    - group 2 consisting of SEQ ID NO.: 3 - 5 and variants thereof,
    - group 3 consisting of SEQ ID NO.: 6 - 8 and variants thereof,
    - group 4 consisting of SEQ ID NO.: 9 - 11 and variants thereof,
    - group 5 consisting of SEQ ID NO.: 12 - 18 and variants thereof, and
    - group 6 consisting of SEQ ID NO.: 19 - 23 and variants thereof, wherein each variant differs from the original peptide in 15 % or less of the amino acids,

- applying the R-peptides to a chromatography column, and
- determining the retention time of each peptide,

wherein each variant does not comprise methionine, tryptophan, cysteine, an aspartic acid-proline motif, an asparagine-proline motif or an asparagine-glycine motif, and
wherein each amino acid that differs from the original peptide is obtained by a conservative amino acid exchange.

**11.** The method of claim 10, wherein the chromatography is performed using a reverse-phase liquid chromatography instrument, preferably a HPLC instrument, more preferred a low pH reverse-phase liquid chromatography mass spectrometry (RP-LC-MS) instrument.

**12.** The method of claim 10, wherein reverse-phase chromatography is followed by selected reaction monitoring mass spectrometry.

**13.** A method for estimating a retention time of a target peptide during a liquid chromatography run, comprising the steps

- providing at least 8 reference peptides (R-peptides) selected from the group consisting of SEQ ID NO.: 1 - 27 and variants thereof, wherein at least one R-peptide is selected from each group comprising

    - group 2 consisting of SEQ ID NO.: 3 - 5 and variants thereof,
    - group 3 consisting of SEQ ID NO.: 6 - 8 and variants thereof,
    - group 4 consisting of SEQ ID NO.: 9 - 11 and variants thereof,
    - group 5 consisting of SEQ ID NO.: 12 - 18 and variants thereof, and
    - group 6 consisting of SEQ ID NO.: 19 - 23 and variants thereof, wherein each variant differs from the original peptide in 15 % or less of the amino acids,

- adding the peptides to a sample comprising the target peptide,
- applying the sample to a chromatography column,
- determining the retention times of the R-peptides, and
- estimating the retention time of the target peptide according to the determined retention times of the R-peptides,

wherein each variant does not comprise methionine, tryptophan, cysteine, an aspartic acid-proline motif, an asparagine-proline motif or an asparagine-glycine motif, and
wherein each amino acid that differs from the original peptide is obtained by a conservative amino acid exchange.

**Patentansprüche**

1. Zusammensetzung umfassend mindestens 8 Referenzpeptide (R-Peptide), ausgewählt aus der Gruppe, bestehend aus SEQ ID NO.: 1 - 27 und Varianten davon, wobei mindestens ein R-Peptid aus jeder Gruppe ausgewählt ist, umfassend

   - Gruppe 2 bestehend aus SEQ ID NO.: 3 - 5 und Varianten davon,
   - Gruppe 3 bestehend aus SEQ ID NO.: 6 - 8 und Varianten davon,
   - Gruppe 4 bestehend aus SEQ ID NO.: 9 - 11 und Varianten davon,
   - Gruppe 5, bestehend aus SEQ ID NO.: 12 - 18 und Varianten davon, und
   - Gruppe 6 bestehend aus SEQ ID NO.: 19 - 23 und Varianten davon,

   wobei sich jede Variante vom ursprünglichen Peptid in 15 % oder weniger der Aminosäuren unterscheidet, wobei jede Variante nicht Methionin, Tryptophan, Cystein, ein Asparaginsäure-Prolin-Motiv, ein Asparagin-Prolin-Motiv oder ein Asparagin-Glycin-Motiv umfasst, und
   wobei jede Aminosäure, die sich von dem ursprünglichen Peptid unterscheidet, durch einen konservativen Aminosäureaustausch erhalten wird.

2. Zusammensetzung nach Anspruch 1, wobei die Zusammensetzung mindestens 10, vorzugsweise mindestens 15, weiter bevorzugt mindestens 20, am meisten bevorzugt 27 R-Peptide umfasst.

3. Zusammensetzung nach Anspruch 1 oder 2, wobei mindestens ein R-Peptid aus der Gruppe 7, bestehend aus SEQ ID NO.: 24 - 27, ausgewählt ist.

4. Zusammensetzung nach einem der Ansprüche 1 bis 3, wobei mindestens ein R-Peptid aus der Gruppe 1, bestehend aus SEQ ID NO.: 1 und 2, ausgewählt ist.

5. Polypeptid umfassend mindestens 8 Referenzpeptide (R-Peptide), ausgewählt aus der Gruppe, bestehend aus SEQ ID NO.: 1 - 27 und Varianten davon, wobei mindestens ein R-Peptid aus jeder Gruppe ausgewählt ist, umfassend

   - Gruppe 2 bestehend aus SEQ ID NO.: 3 - 5 und Varianten davon,
   - Gruppe 3 bestehend aus SEQ ID NO.: 6 - 8 und Varianten davon,
   - Gruppe 4 bestehend aus SEQ ID NO.: 9 - 11 und Varianten davon,
   - Gruppe 5, bestehend aus SEQ ID NO.: 12 - 18 und Varianten davon, und
   - Gruppe 6 bestehend aus SEQ ID NO.: 19 - 23 und Varianten davon,

   wobei sich jede Variante vom ursprünglichen Peptid in 15 % oder weniger der Aminosäuren unterscheidet, wobei jede Variante nicht Methionin, Tryptophan, Cystein, ein Asparaginsäure-Prolin-Motiv, ein Asparagin-Prolin-Motiv oder ein Asparagin-Glycin-Motiv umfasst, und
   wobei jede Aminosäure, die sich von dem ursprünglichen Peptid unterscheidet, durch einen konservativen Aminosäureaustausch erhalten wird.

6. Polypeptid nach Anspruch 5, wobei das Polypeptid jedes R-Peptid in einer Stöchiometrie von 1:1 umfasst.

7. Kit umfassend mindestens 8 Referenzpeptide (R-Peptide), ausgewählt aus der Gruppe, bestehend aus SEQ ID NO.: 1 - 27 und Varianten davon, wobei mindestens ein R-Peptid aus jeder Gruppe ausgewählt ist, umfassend

   - Gruppe 2 bestehend aus SEQ ID NO.: 3 - 5 und Varianten davon,
   - Gruppe 3 bestehend aus SEQ ID NO.: 6 - 8 und Varianten davon,
   - Gruppe 4 bestehend aus SEQ ID NO.: 9 - 11 und Varianten davon,
   - Gruppe 5, bestehend aus SEQ ID NO.: 12 - 18 und Varianten davon, und
   - Gruppe 6 bestehend aus SEQ ID NO.: 19 - 23 und Varianten davon,

   wobei sich jede Variante vom ursprünglichen Peptid in 15 % oder weniger der Aminosäuren unterscheidet, wobei jede Variante nicht Methionin, Tryptophan, Cystein, ein Asparaginsäure-Prolin-Motiv, ein Asparagin-Prolin-Motiv oder ein Asparagin-Glycin-Motiv umfasst, und
   wobei jede Aminosäure, die sich von dem ursprünglichen Peptid unterscheidet, durch einen konservativen Aminosäureaustausch erhalten wird.

8. Verwendung von mindestens 8 Referenzpeptiden (R-Peptide), ausgewählt aus der Gruppe bestehend aus SEQ ID NO.: 1 - 27 und Varianten davon, wobei mindestens ein R-Peptid aus jeder Gruppe ausgewählt ist, umfassend

- Gruppe 2 bestehend aus SEQ ID NO.: 3 - 5 und Varianten davon,
- Gruppe 3 bestehend aus SEQ ID NO.: 6 - 8 und Varianten davon,
- Gruppe 4 bestehend aus SEQ ID NO.: 9 - 11 und Varianten davon,
- Gruppe 5, bestehend aus SEQ ID NO.: 12 - 18 und Varianten davon, und
- Gruppe 6 bestehend aus SEQ ID NO.: 19 - 23 und Varianten davon,

wobei sich jede Variante vom ursprünglichen Peptid in 15 % oder weniger der Aminosäuren unterscheidet, für Umkehrphasen-Flüssigkeitschromatographie, wobei jede Variante nicht Methionin, Tryptophan, Cystein, ein Asparaginsäure-Prolin-Motiv, ein Asparagin-Prolin-Motiv oder ein Asparagin-Glycin-Motiv umfasst, und wobei jede Aminosäure, die sich von dem ursprünglichen Peptid unterscheidet, durch einen konservativen Aminosäureaustausch erhalten wird.

**9.** Verwendung von mindestens 8 Referenzpeptiden (R-Peptide), ausgewählt aus der Gruppe, bestehend aus SEQ ID NO.: 1 - 27 und Varianten davon, wobei mindestens ein R-Peptid aus jeder Gruppe ausgewählt ist, umfassend

- Gruppe 2 bestehend aus SEQ ID NO.: 3 - 5 und Varianten davon,
- Gruppe 3 bestehend aus SEQ ID NO.: 6 - 8 und Varianten davon,
- Gruppe 4 bestehend aus SEQ ID NO.: 9 - 11 und Varianten davon,
- Gruppe 5, bestehend aus SEQ ID NO.: 12 - 18 und Varianten davon, und
- Gruppe 6 bestehend aus SEQ ID NO.: 19 - 23 und Varianten davon,

wobei sich jede Variante vom ursprünglichen Peptid in 15 % oder weniger der Aminosäuren unterscheidet, zur ausgewählten Reaktionsüberwachung (SRM), wobei jede Variante nicht Methionin, Tryptophan, Cystein, ein Asparaginsäure-Prolin-Motiv, ein Asparagin-Prolin-Motiv oder ein Asparagin-Glycin-Motiv umfasst, und wobei jede Aminosäure, die sich von dem ursprünglichen Peptid unterscheidet, durch einen konservativen Aminosäureaustausch erhalten wird.

**10.** Verfahren zur Überwachung von Umkehrphasen-Chromatographie, umfassend die Schritte

- Bereitstellen von mindestens 8 Referenzpeptiden (R-Peptide), ausgewählt aus der Gruppe, bestehend aus SEQ ID NO.: 1 - 27 und Varianten davon, wobei mindestens ein R-Peptid aus jeder Gruppe ausgewählt ist, umfassend
- Gruppe 2 bestehend aus SEQ ID NO.: 3 - 5 und Varianten davon,
- Gruppe 3 bestehend aus SEQ ID NO.: 6 - 8 und Varianten davon,
- Gruppe 4 bestehend aus SEQ ID NO.: 9 - 11 und Varianten davon,
- Gruppe 5, bestehend aus SEQ ID NO.: 12 - 18 und Varianten davon, und
- Gruppe 6 bestehend aus SEQ ID NO.: 19 - 23 und Varianten davon, wobei sich jede Variante vom ursprünglichen Peptid in 15 % oder weniger der Aminosäuren unterscheidet,
- Aufbringen der R-Peptide auf eine Chromatographiesäule, und
- Bestimmen der Retentionszeit jedes Peptids,

wobei jede Variante nicht Methionin, Tryptophan, Cystein, ein Asparaginsäure-Prolin-Motiv, ein Asparagin-Prolin-Motiv oder ein Asparagin-Glycin-Motiv umfasst, und wobei jede Aminosäure, die sich von dem ursprünglichen Peptid unterscheidet, durch einen konservativen Aminosäureaustausch erhalten wird.

**11.** Verfahren nach Anspruch 10, wobei die Chromatographie unter Verwendung eines Umkehrphasen-Flüssigkeitschromatographie-Instruments, vorzugsweise eines HPLC-Instruments, weiter bevorzugt eines Umkehrphasen-Flüssigkeitschromatographie-Massenspektrometrie-Instruments mit niedrigem pH-Wert (RP-LC-MS), durchgeführt wird.

**12.** Verfahren nach Anspruch 10, wobei die Umkehrphasen-Chromatographie von ausgewählter Reaktionsüberwachungs-Massenspektrometrie gefolgt wird.

**13.** Verfahren zum Abschätzen einer Retentionszeit eines Zielpeptids während eines Flüssigchromatographie-Laufs, umfassend die Schritte

- Bereitstellen von mindestens 8 Referenzpeptiden (R-Peptide), ausgewählt aus der Gruppe, bestehend aus SEQ ID NO.: 1 - 27 und Varianten davon, wobei mindestens ein R-Peptid aus jeder Gruppe ausgewählt ist, umfassend
- Gruppe 2 bestehend aus SEQ ID NO.: 3 - 5 und Varianten davon,
- Gruppe 3 bestehend aus SEQ ID NO.: 6 - 8 und Varianten davon,

- Gruppe 4 bestehend aus SEQ ID NO.: 9 - 11 und Varianten davon,
- Gruppe 5, bestehend aus SEQ ID NO.: 12 - 18 und Varianten davon, und
- Gruppe 6 bestehend aus SEQ ID NO.: 19 - 23 und Varianten davon, wobei sich jede Variante vom ursprünglichen Peptid in 15 % oder weniger der Aminosäuren unterscheidet,
- Zugeben der Peptide zu einer Probe, die das Zielpeptid umfasst,
- Aufbringen der Probe auf eine Chromatographiesäule,
- Bestimmen der Retentionszeiten der R-Peptide, und
- Abschätzen der Retentionszeit des Zielpeptids entsprechend den ermittelten Retentionszeiten der R-Peptide,

wobei jede Variante nicht Methionin, Tryptophan, Cystein, ein Asparaginsäure-Prolin-Motiv, ein Asparagin-Prolin-Motiv oder ein Asparagin-Glycin-Motiv umfasst, und
wobei jede Aminosäure, die sich von dem ursprünglichen Peptid unterscheidet, durch einen konservativen Aminosäureaustausch erhalten wird.

## Revendications

**1.** Composition comprenant au moins 8 peptides de référence (R-peptides) choisis dans le groupe constitué de SEQ ID N° : 1 à 27 et des variants de ceux-ci, dans laquelle au moins un R-peptide est choisi dans chaque groupe comprenant

- groupe 2 constitué de SEQ ID N° : 3 à 5 et des variants de ceux-ci,
- groupe 3 constitué de SEQ ID N° : 6 à 8 et des variants de ceux-ci,
- groupe 4 constitué de SEQ ID N° : 9 à 11 et des variants de ceux-ci,
- groupe 5 constitué de SEQ ID N° : 12 à 18 et des variants de ceux-ci, et
- groupe 6 constitué de SEQ ID N° : 19 à 23 et des variants de ceux-ci,

dans laquelle chaque variant diffère du peptide d'origine dans 15 % ou moins des acides aminés,
dans laquelle chaque variant ne comprend pas la méthionine, le tryptophane, la cystéine, un motif acide aspartique-proline, un motif asparagine-proline ou un motif asparagine-glycine, et
dans laquelle chaque acide aminé qui diffère du peptide d'origine est obtenu par un échange d'acide aminé conservateur.

**2.** Composition selon la revendication 1, dans laquelle la composition comprend au moins 10, de préférence au moins 15, de manière davantage préférée au moins 20, de manière préférée entre toutes 27 R-peptides.

**3.** Composition selon la revendication 1 ou la revendication 2, dans laquelle au moins un R-peptide est choisi dans le groupe 7 constitué de SEQ ID N° : 24 à 27.

**4.** Composition selon l'une quelconque des revendications 1 à 3, dans laquelle au moins un R-peptide est choisi dans le groupe 1 constitué de SEQ ID N° : 1 et 2.

**5.** Polypeptide comprenant au moins 8 peptides de référence (R-peptides) choisis dans le groupe constitué de SEQ ID N° : 1 à 27 et des variants de ceux-ci, dans lequel au moins un R-peptide est choisi dans chaque groupe comprenant

- groupe 2 constitué de SEQ ID N° : 3 à 5 et des variants de ceux-ci,
- groupe 3 constitué de SEQ ID N° : 6 à 8 et des variants de ceux-ci,
- groupe 4 constitué de SEQ ID N° : 9 à 11 et des variants de ceux-ci,
- groupe 5 constitué de SEQ ID N° : 12 à 18 et des variants de ceux-ci, et
- groupe 6 constitué de SEQ ID N° : 19 à 23 et des variants de ceux-ci,

dans lequel chaque variant diffère du peptide d'origine dans 15 % ou moins des acides aminés,
dans lequel chaque variant ne comprend pas la méthionine, le tryptophane, la cystéine, un motif acide aspartique-proline, un motif asparagine-proline ou un motif asparagine-glycine, et
dans lequel chaque acide aminé qui diffère du peptide d'origine est obtenu par un échange d'acide aminé conservateur.

**6.** Polypeptide selon la revendication 5, dans lequel le polypeptide comprend chaque R-peptide dans une stœchiométrie de 1 : 1.

**7.** Kit comprenant au moins 8 peptides de référence (R-peptides) choisis dans le groupe constitué de SEQ ID N° : 1 à 27 et des variants de ceux-ci, dans lequel au moins un R-peptide est choisi dans chaque groupe comprenant

- groupe 2 constitué de SEQ ID N° : 3 à 5 et des variants de ceux-ci,
- groupe 3 constitué de SEQ ID N° : 6 à 8 et des variants de ceux-ci,
- groupe 4 constitué de SEQ ID N° : 9 à 11 et des variants de ceux-ci,
- groupe 5 constitué de SEQ ID N° : 12 à 18 et des variants de ceux-ci, et
- groupe 6 constitué de SEQ ID N° : 19 à 23 et des variants de ceux-ci,

dans lequel chaque variant diffère du peptide d'origine dans 15 % ou moins des acides aminés,
dans lequel chaque variant ne comprend pas la méthionine, le tryptophane, la cystéine, un motif acide aspartique-proline, un motif asparagine-proline ou un motif asparagine-glycine, et
dans lequel chaque acide aminé qui diffère du peptide d'origine est obtenu par un échange d'acide aminé conservateur.

**8.** Utilisation d'au moins 8 peptides de référence (R-peptides) choisis dans le groupe constitué de SEQ ID N° : 1 à 27 et des variants de ceux-ci, dans laquelle au moins un R-peptide est choisi dans chaque groupe comprenant

- groupe 2 constitué de SEQ ID N° : 3 à 5 et des variants de ceux-ci,
- groupe 3 constitué de SEQ ID N° : 6 à 8 et des variants de ceux-ci,
- groupe 4 constitué de SEQ ID N° : 9 à 11 et des variants de ceux-ci,
- groupe 5 constitué de SEQ ID N° : 12 à 18 et des variants de ceux-ci, et
- groupe 6 constitué de SEQ ID N° : 19 à 23 et des variants de ceux-ci,

dans laquelle chaque variant diffère du peptide d'origine dans 15 % ou moins des acides aminés, pour une chromatographie en phase inverse liquide,
dans laquelle chaque variant ne comprend pas la méthionine, le tryptophane, la cystéine, un motif acide aspartique-proline, un motif asparagine-proline ou un motif asparagine-glycine, et
dans laquelle chaque acide aminé qui diffère du peptide d'origine est obtenu par un échange d'acide aminé conservateur.

**9.** Utilisation d'au moins 8 peptides de référence (R-peptides) choisis dans le groupe constitué de SEQ ID N° : 1 à 27 et des variants de ceux-ci, dans laquelle au moins un R-peptide est choisi dans chaque groupe comprenant

- groupe 2 constitué de SEQ ID N° : 3 à 5 et des variants de ceux-ci,
- groupe 3 constitué de SEQ ID N° : 6 à 8 et des variants de ceux-ci,
- groupe 4 constitué de SEQ ID N° : 9 à 11 et des variants de ceux-ci,
- groupe 5 constitué de SEQ ID N° : 12 à 18 et des variants de ceux-ci, et
- groupe 6 constitué de SEQ ID N° : 19 à 23 et des variants de ceux-ci,

dans laquelle chaque variant diffère du peptide d'origine dans 15 % ou moins des acides aminés, pour une surveillance de réaction sélectionnée (SRM),
dans laquelle chaque variant ne comprend pas la méthionine, le tryptophane, la cystéine, un motif acide aspartique-proline, un motif asparagine-proline ou un motif asparagine-glycine, et
dans laquelle chaque acide aminé qui diffère du peptide d'origine est obtenu par un échange d'acide aminé conservateur.

**10.** Procédé de surveillance d'une chromatographie en phase inverse, comprenant les étapes consistant à

- fournir au moins 8 peptides de référence (R-peptides) choisis dans le groupe constitué de SEQ ID N° : 1 à 27 et des variants de ceux-ci, dans lequel au moins un R-peptide est choisi dans chaque groupe comprenant
- groupe 2 constitué de SEQ ID N° : 3 à 5 et des variants de ceux-ci,
- groupe 3 constitué de SEQ ID N° : 6 à 8 et des variants de ceux-ci,
- groupe 4 constitué de SEQ ID N° : 9 à 11 et des variants de ceux-ci,
- groupe 5 constitué de SEQ ID N° : 12 à 18 et des variants de ceux-ci, et
- groupe 6 constitué de SEQ ID N° : 19 à 23 et des variants de ceux-ci,

dans lequel chaque variant diffère du peptide d'origine dans 15 % ou moins des acides aminés,
- appliquer les R-peptides à une colonne de chromatographie, et
- déterminer le temps de rétention de chaque peptide,

dans lequel chaque variant ne comprend pas la méthionine, le tryptophane, la cystéine, un motif acide aspartique-proline, un motif asparagine-proline ou un motif asparagine-glycine, et
dans lequel chaque acide aminé qui diffère du peptide d'origine est obtenu par un échange d'acide aminé conservateur.

11. Procédé selon la revendication 10, dans lequel la chromatographie est réalisée en utilisant un instrument de chromatographie en phase inverse liquide, de préférence un instrument de HPLC, de manière davantage préférée, un instrument de spectrométrie de masse - chromatographie en phase inverse liquide à pH bas (RP-LC-MS).

12. Procédé selon la revendication 10, dans lequel la chromatographie en phase inverse est suivie d'une spectrométrie de masse avec surveillance de réaction sélectionnée.

13. Procédé d'estimation d'un temps de rétention d'un peptide cible pendant un cycle de chromatographie en phase liquide, comprenant les étapes consistant à

- fournir au moins 8 peptides de référence (R-peptides) choisis dans le groupe constitué de SEQ ID N° : 1 à 27 et des variants de ceux-ci, dans lequel au moins un R-peptide est choisi dans chaque groupe comprenant
- groupe 2 constitué de SEQ ID N° : 3 à 5 et des variants de ceux-ci,
- groupe 3 constitué de SEQ ID N° : 6 à 8 et des variants de ceux-ci,
- groupe 4 constitué de SEQ ID N° : 9 à 11 et des variants de ceux-ci,
- groupe 5 constitué de SEQ ID N° : 12 à 18 et des variants de ceux-ci, et
- groupe 6 constitué de SEQ ID N° : 19 à 23 et des variants de ceux-ci,

dans lequel chaque variant diffère du peptide d'origine dans 15 % ou moins des acides aminés,

- ajouter les peptides à un échantillon comprenant le peptide cible,
- appliquer l'échantillon à une colonne de chromatographie,
- déterminer les temps de rétention des R-peptides, et
- estimer le temps de rétention du peptide cible selon les temps de rétention déterminés des R-peptides,

dans lequel chaque variant ne comprend pas la méthionine, le tryptophane, la cystéine, un motif acide aspartique-proline, un motif asparagine-proline ou un motif asparagine-glycine, et

dans lequel chaque acide aminé qui diffère du peptide d'origine est obtenu par un échange d'acide aminé conservateur.

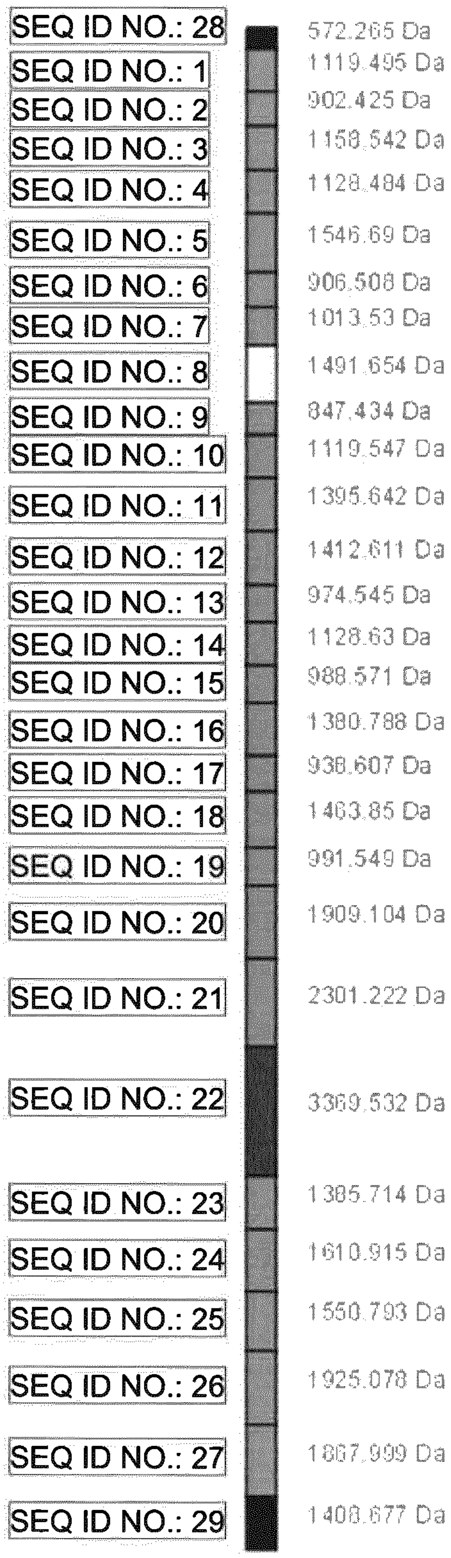
SEQ ID NO.: 28
572.265 Da
SEQ ID NO.: 1
1119.495 Da
SEQ ID NO.: 2
902.425 Da
SEQ ID NO.: 3
1158.542 Da
SEQ ID NO.: 4
1128.484 Da
SEQ ID NO.: 5
1546.69 Da
SEQ ID NO.: 6
906.508 Da
SEQ ID NO.: 7
1013.53 Da
SEQ ID NO.: 8
1491.654 Da
SEQ ID NO.: 9
847.434 Da
SEQ ID NO.: 10
1119.547 Da
SEQ ID NO.: 11
1395.642 Da
SEQ ID NO.: 12
1412.611 Da
SEQ ID NO.: 13
974.545 Da
SEQ ID NO.: 14
1128.63 Da
SEQ ID NO.: 15
988.571 Da
SEQ ID NO.: 16
1380.788 Da
SEQ ID NO.: 17
938.607 Da
SEQ ID NO.: 18
1463.85 Da
SEQ ID NO.: 19
991.549 Da
SEQ ID NO.: 20
1909.104 Da
SEQ ID NO.: 21
2301.222 Da
SEQ ID NO.: 22
3369.532 Da
SEQ ID NO.: 23
1385.714 Da
SEQ ID NO.: 24
1610.915 Da
SEQ ID NO.: 25
1550.793 Da
SEQ ID NO.: 26
1925.078 Da
SEQ ID NO.: 27
1867.999 Da
SEQ ID NO.: 29
1408.677 Da

Figure 1

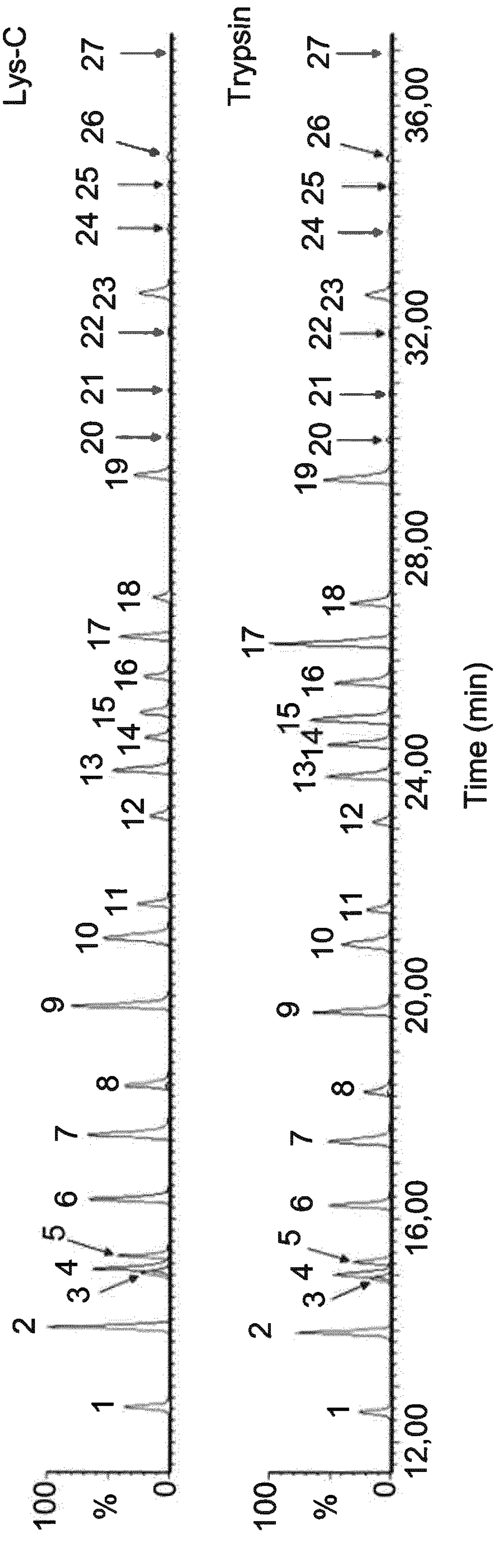
Lys-C
Trypsin
100
%
0
12,00
16,00
20,00
24,00
28,00
32,00
36,00
Time (min)
1 2 3 4 5 6 7 8 9 10 11 12 13 14 15 16 17 18 19 20 21 22 23 24 25 26 27

Figure 2

3/11

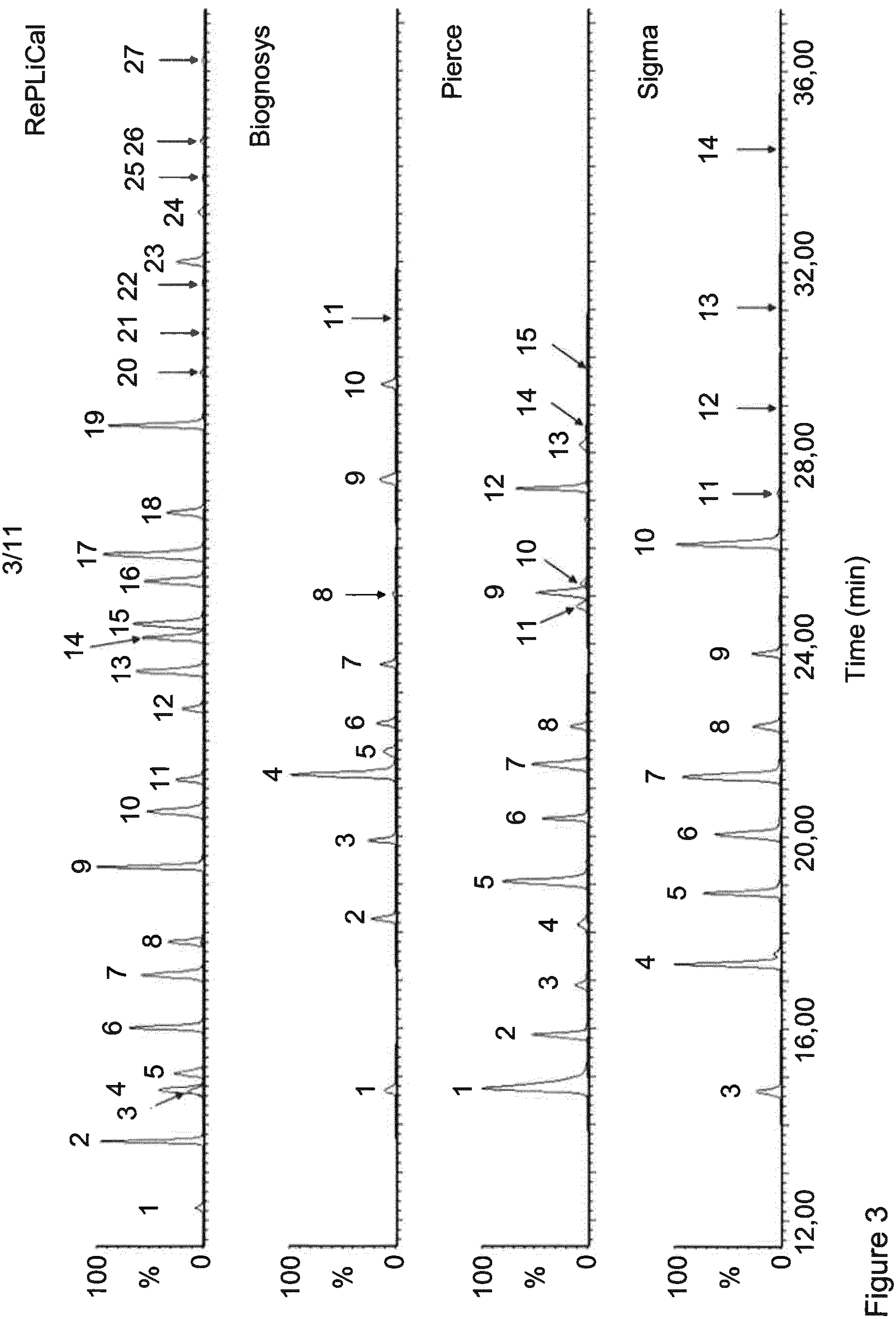
RePLiCal
Biognosys
Pierce
Sigma
100
%
0
12,00
16,00
20,00
24,00
28,00
32,00
36,00
Time (min)

Figure 3

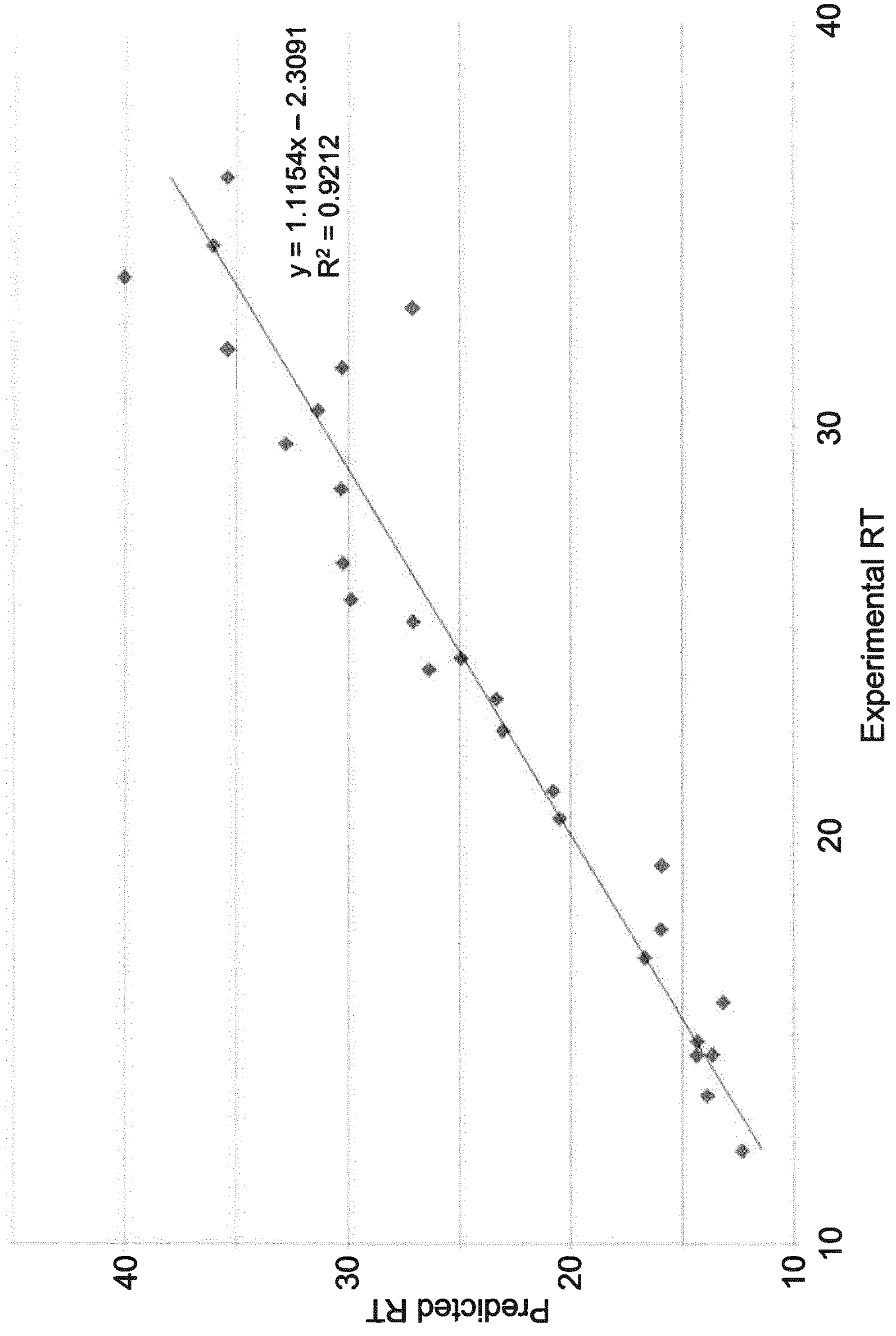
y = 1.1154x – 2.3091
R² = 0.9212
Predicted RT
Experimental RT
10
20
30
40
10
20
30
40

Figure 4

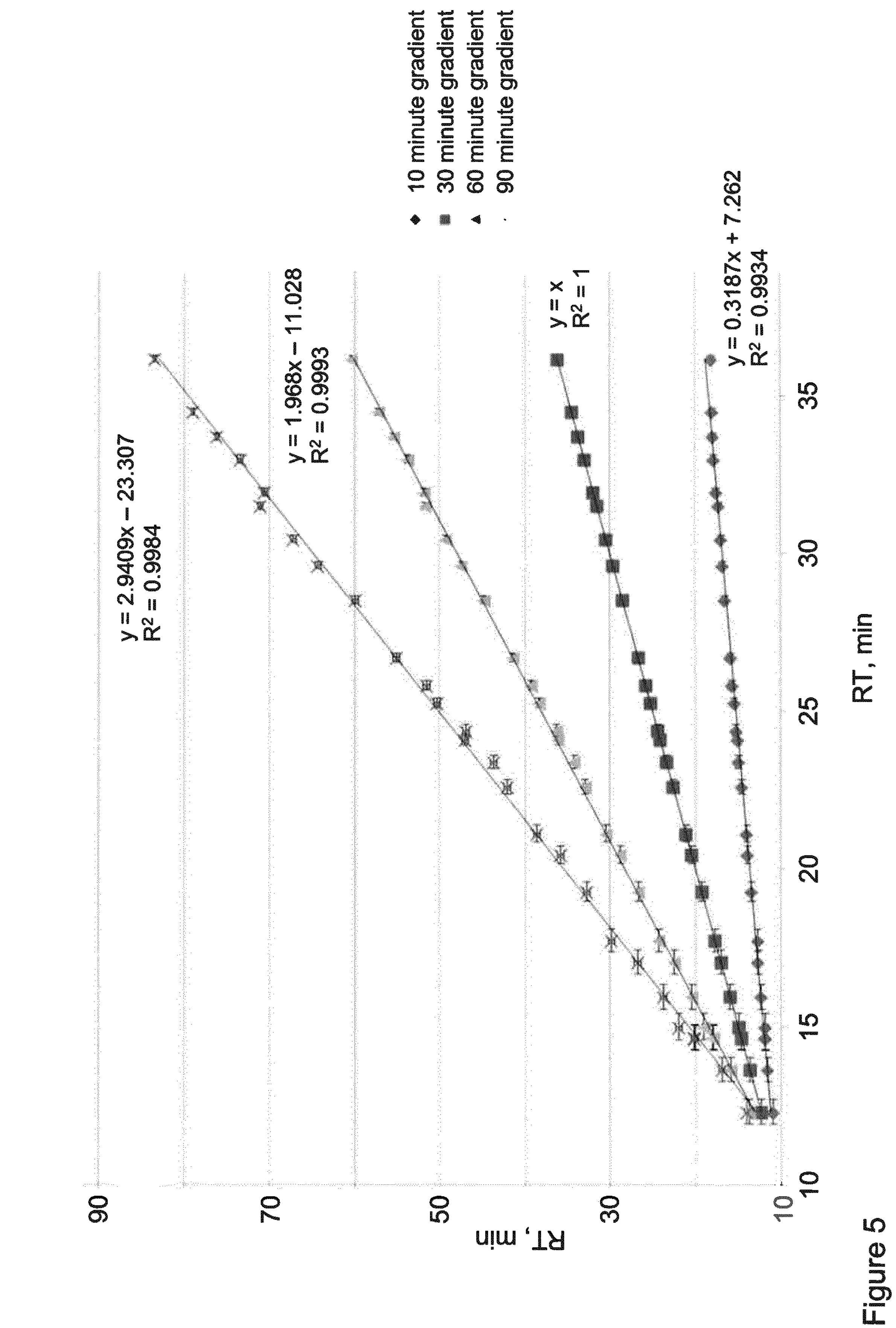
10 minute gradient
30 minute gradient
60 minute gradient
90 minute gradient
y = 2.9409x – 23.307
R² = 0.9984
y = 1.968x – 11.028
R² = 0.9993
y = x
R² = 1
y = 0.3187x + 7.262
R² = 0.9934
RT, min
RT, min
10
30
50
70
90
10
15
20
25
30
35

Figure 5

A

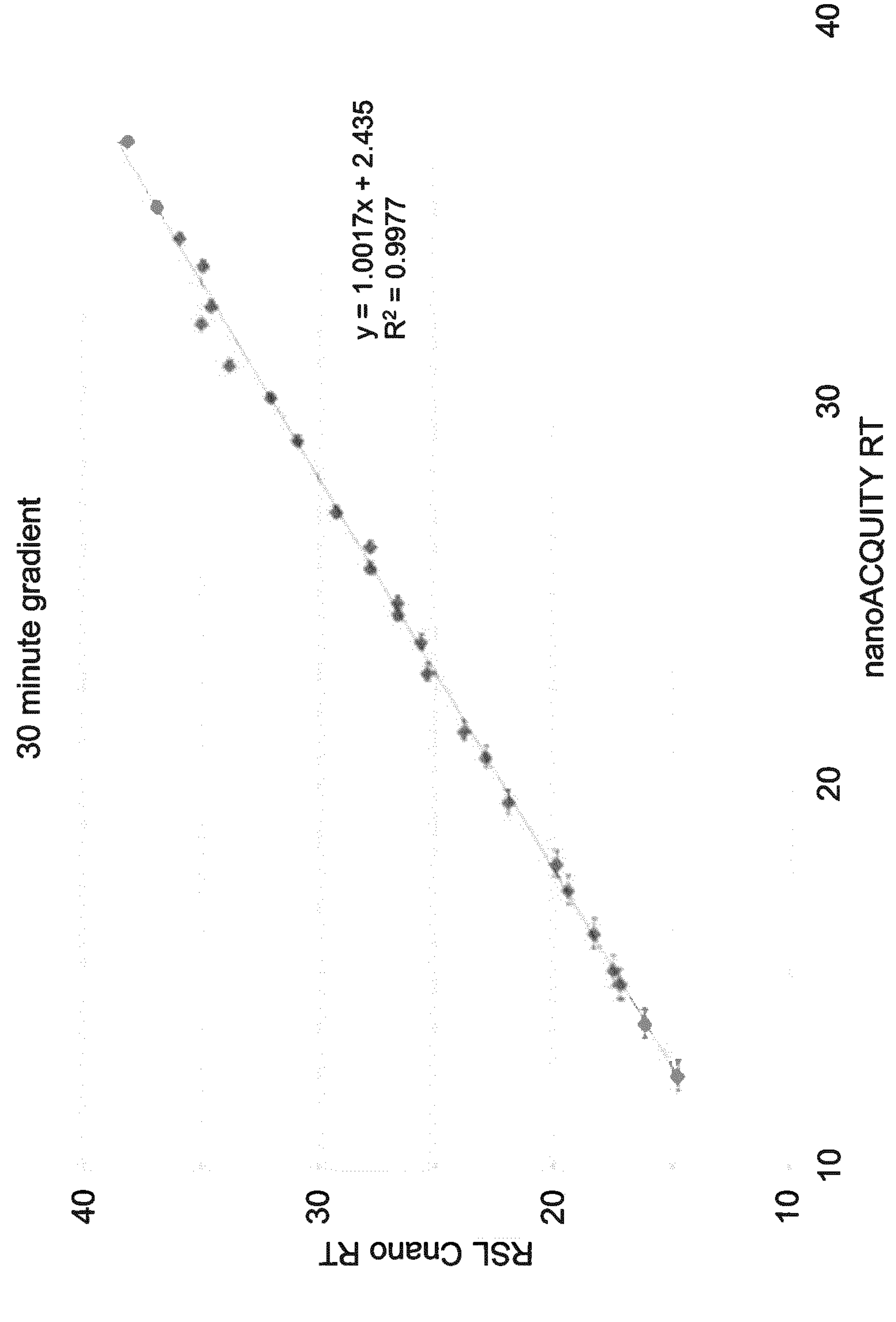
30 minute gradient
y = 1.0017x + 2.435
R² = 0.9977
RSL Cnano RT
40
30
20
10
10
20
30
40
nanoACQUITY RT

Figure 6

B

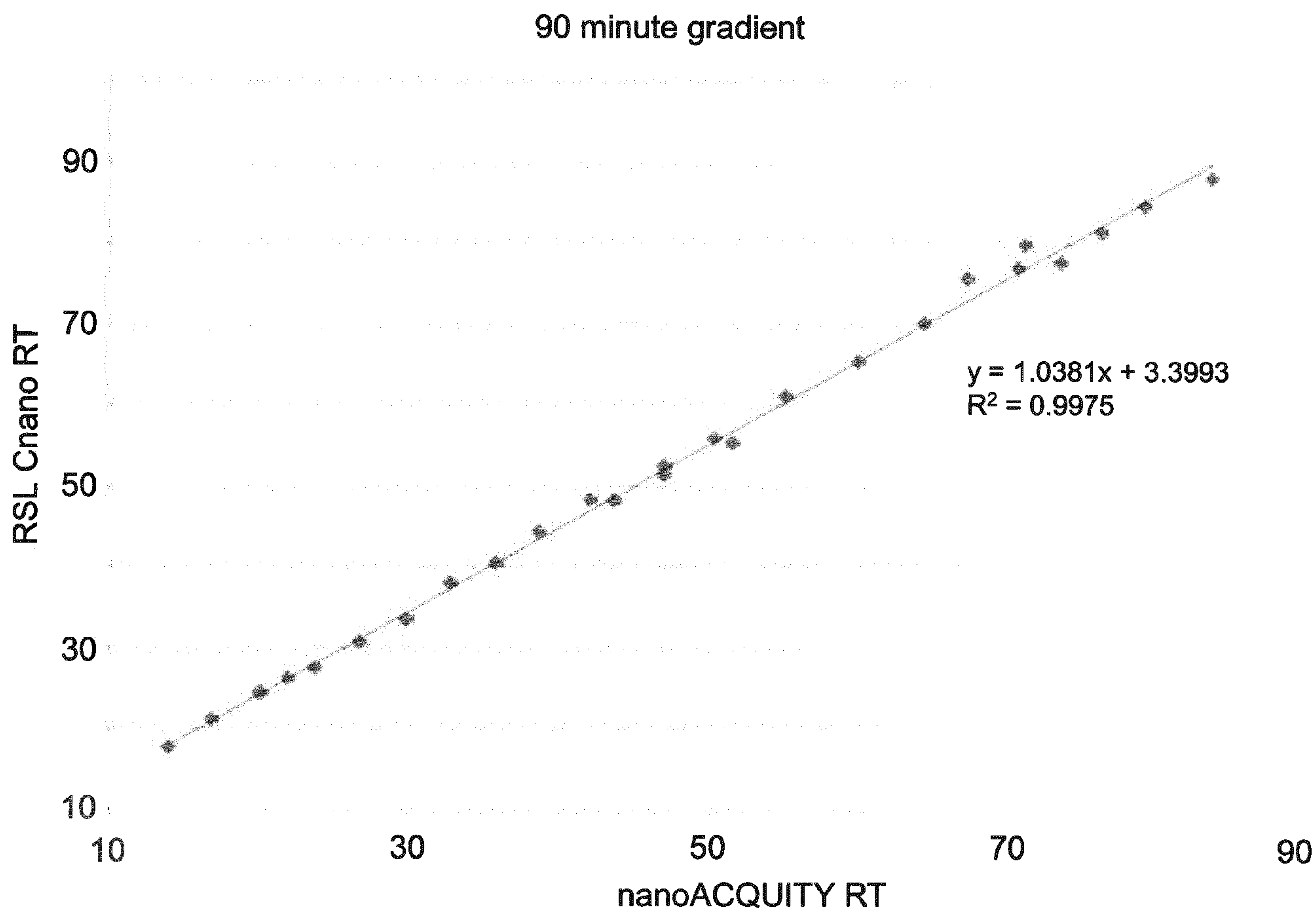


Figure 6 (continued)

8/11

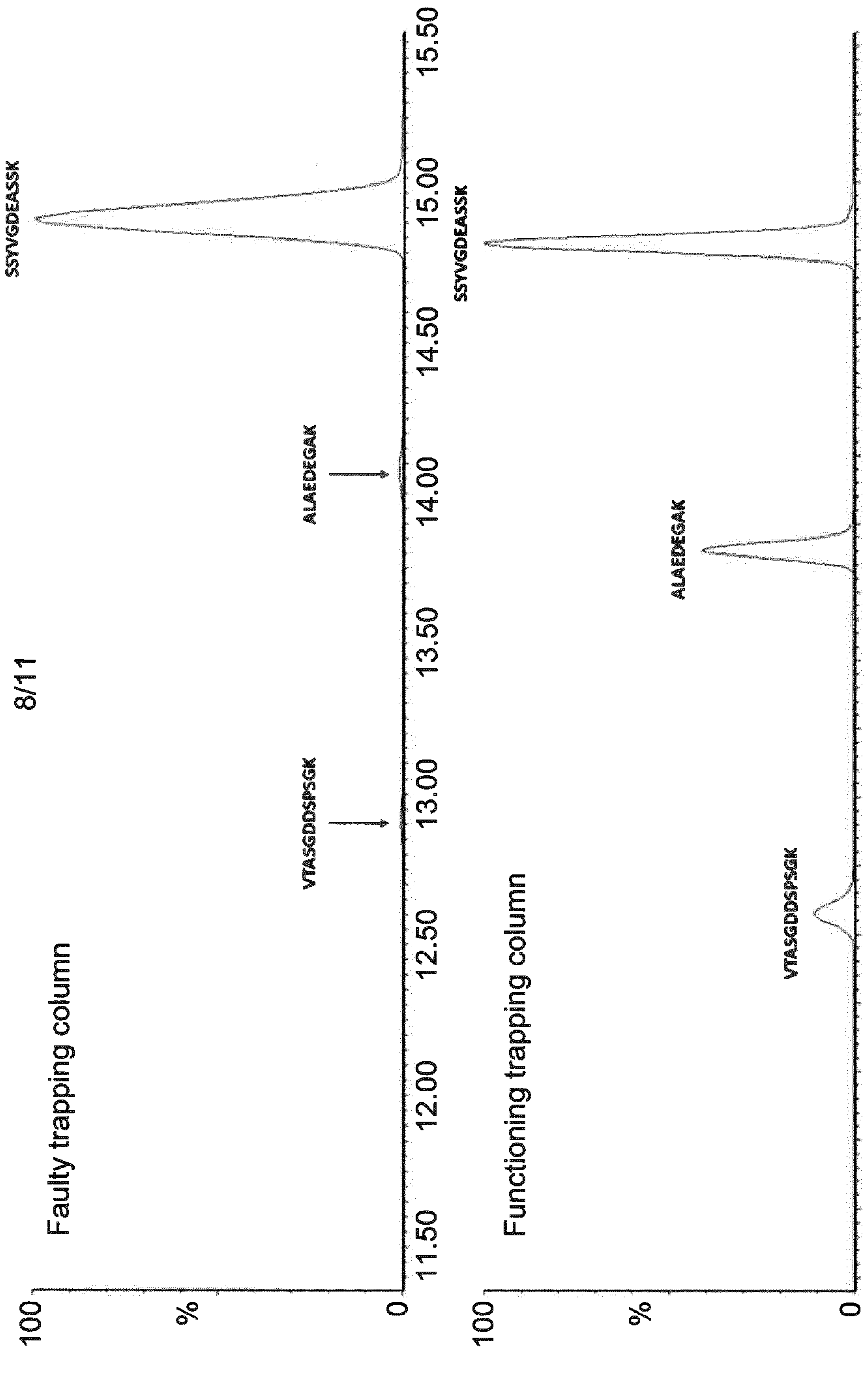

Faulty trapping column
Functioning trapping column
SSYVGDEASSK
ALAEDEGAK
VTASGDDSPSGK
100
%
0
11.50
12.00
12.50
13.00
13.50
14.00
14.50
15.00
15.50


Figure 7

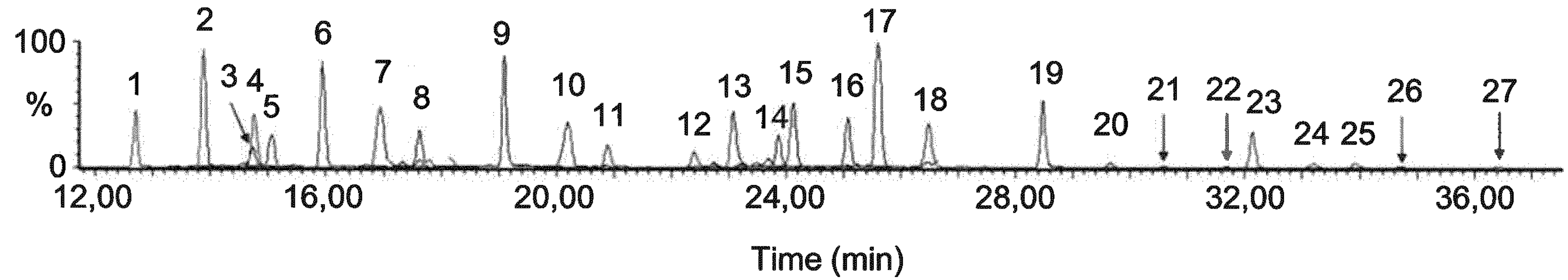
100
%
0
12,00
16,00
20,00
24,00
28,00
32,00
36,00
Time (min)
1
2
3
4
5
6
7
8
9
10
11
12
13
14
15
16
17
18
19
20
21
22
23
24
25
26
27

Figure 8

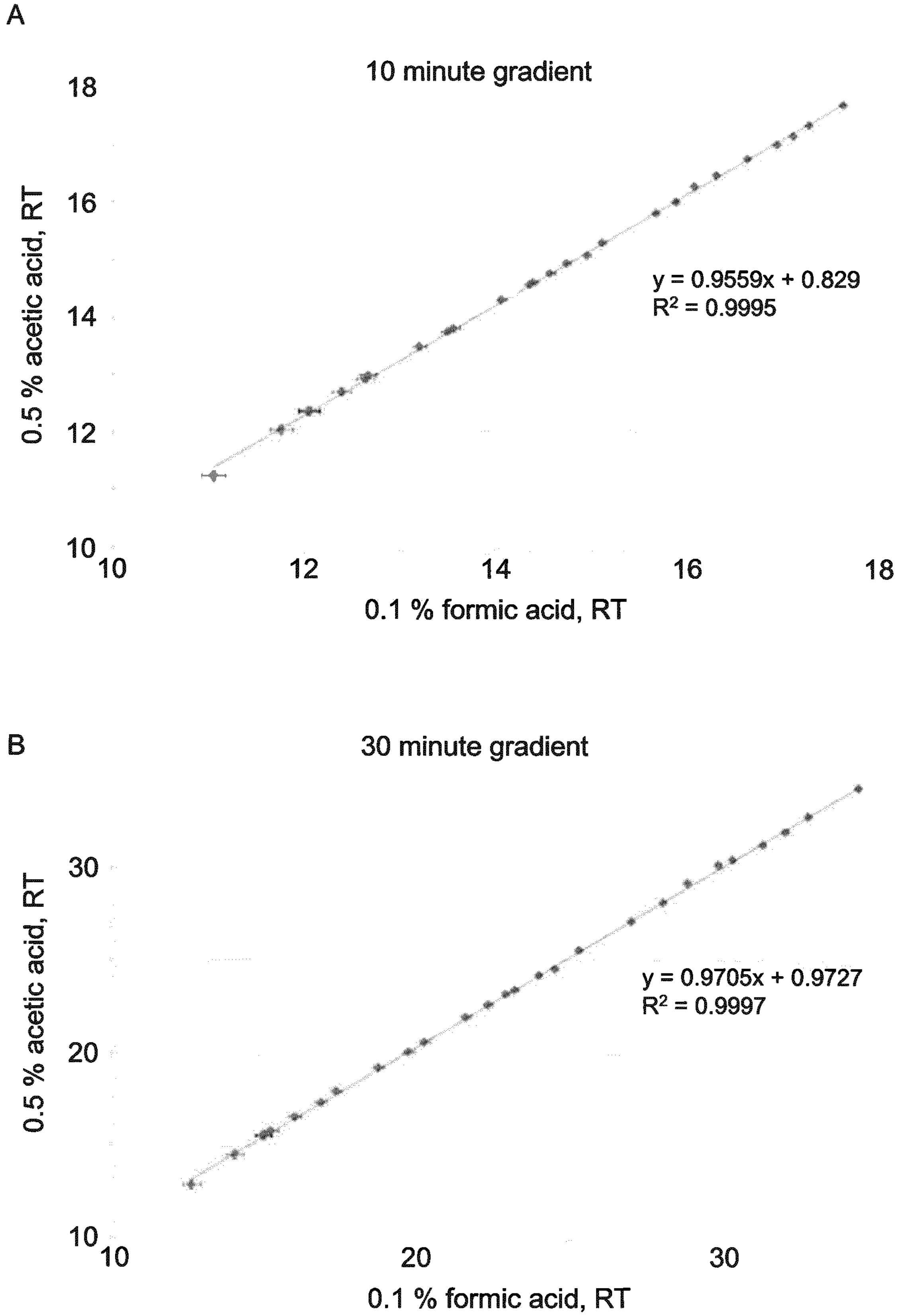
A
10 minute gradient
y = 0.9559x + 0.829
R² = 0.9995
0.5 % acetic acid, RT
0.1 % formic acid, RT
10
12
14
16
18
B
30 minute gradient
y = 0.9705x + 0.9727
R² = 0.9997
0.5 % acetic acid, RT
0.1 % formic acid, RT
10
20
30

Figure 9

C

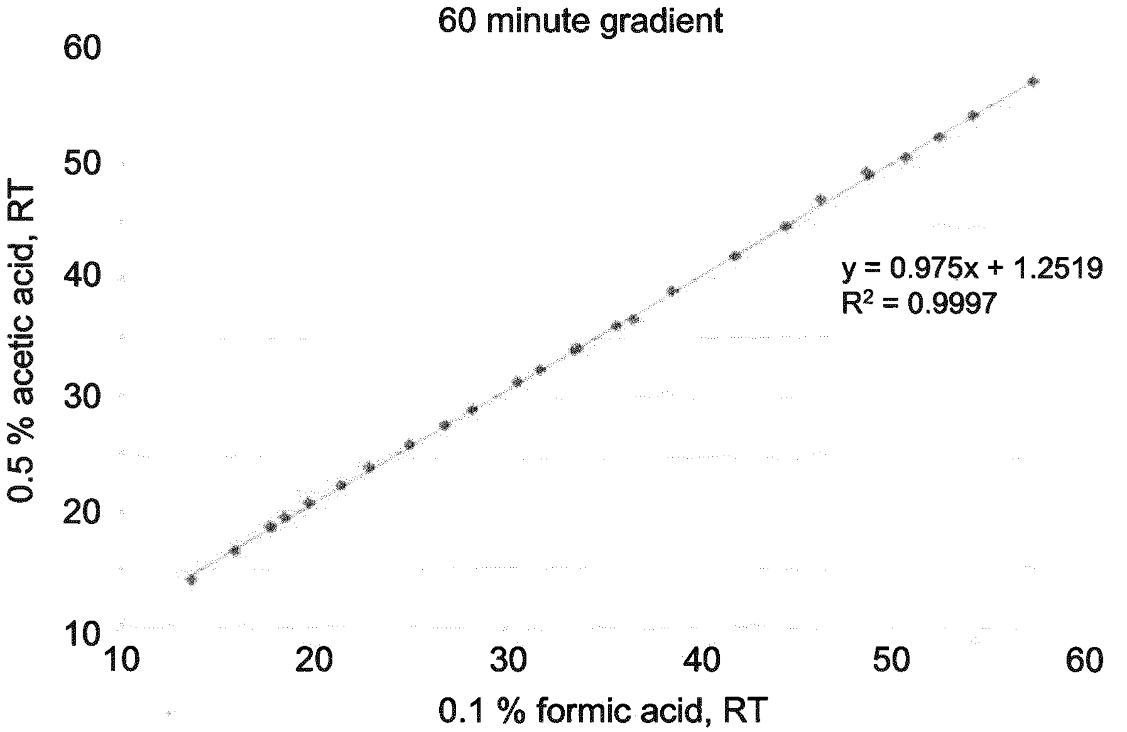


D

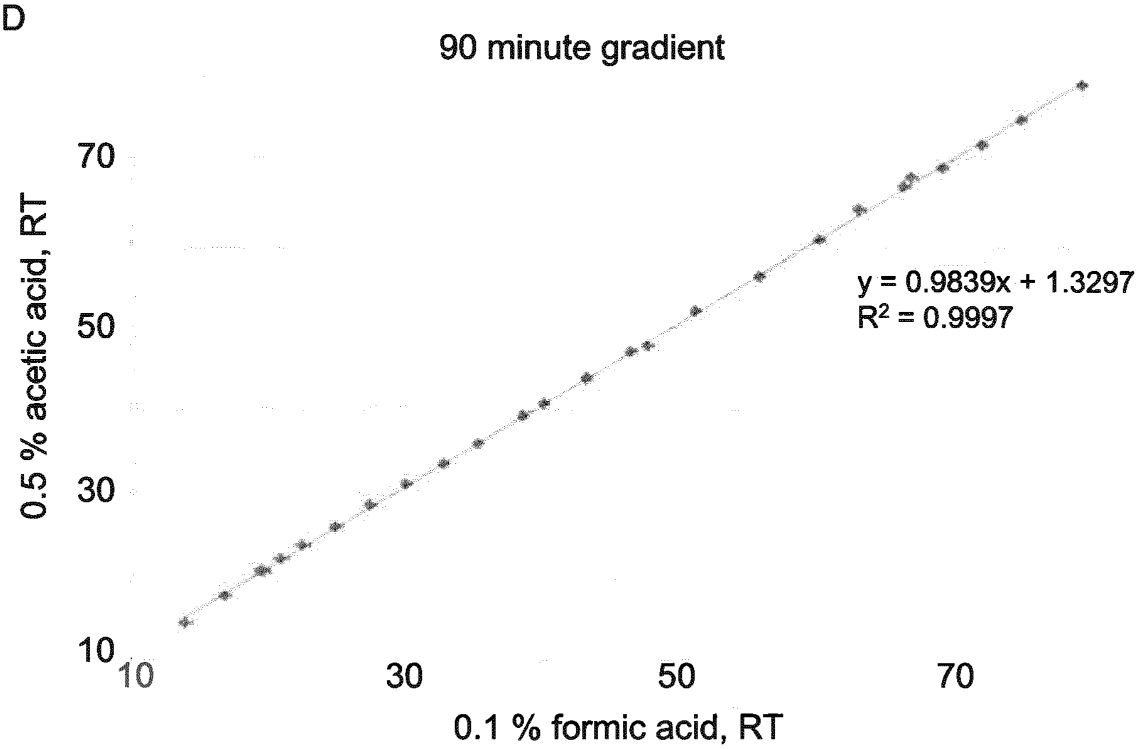


Figure 9 (continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1504820 A

### Non-patent literature cited in the description


- **BURKHART, J. M. ; PREMSLER, T. ; SICKMANN, A.** Quality control of nano-LC-MS systems using stable isotope-coded peptides. *Proteomics,* 2011, vol. 11, 1049-1057
- **ESCHER, C. ; REITER, L. ; MACLEAN, B. ; OSSOLA, R. ; HERZOG, F. ; CHILTON, J. ; MACCOSS, M. J. ; RINNER, O.** Using iRT, a normalized retention time for more targeted measurement of peptides. *Proteomics,* 2012, vol. 12, 1111-1121
- **GALLIEN, S. ; PETERMAN, S. ; KIYONAMI, R. ; SOUADY, J. ; DURIEZ, E. ; SCHOEN, A. ; DOMON, B.** Highly multiplexed targeted proteomics using precise control of peptide retention time. *Proteomics,* 2012, vol. 12, 1122-1133
- **GLAJCH, J. L. ; QUARRY, M. A. ; VASTA, J. F. ; SNYDER, L. R.** Separation of peptide mixtures by reversed-phase gradient elution. Use of flow rate changes for controlling band spacing and improving resolution. *Anal. Chem.,* 1986, vol. 58, 280-285
- **HOLMAN, S. W. ; SIMS, P. F. G. ; EYERS, C. E.** The use of selected reaction monitoring in quantitative proteomics. *Bioanalysis,* 2012, vol. 4, 1763-1786
- **KROKHIN, O. V.** Sequence-specific retention calculator. Algorithm for peptide retention prediction in ion-pair RP-HPLC: Application to 300- and 100-Å pore size C18 sorbents. *Anal. Chem.,* 2006, vol. 78, 7785-7795
- **KROKHIN, O. V. ; SPICER, V.** Peptide retention standards and hydrophobicity indexes in reversed-phase high-performance liquid chromatography of peptides. *Anal. Chem.,* 2009, vol. 81, 9522-9530
- **MALMSTRÖM, L. ; MALMSTRÖM, J. ; SELEVSEK, N. ; ROSENBERGER, G. ; AEBERSOLD, R.** Automated workflow for large-scale selected reaction monitoring experiments. *J. Proteome Res.,* 2012, vol. 11, 1644-1653
- **MIRZAEI, H. ; BRUSNIAK, M.-Y. ; MUELLER, L. N. ; LETARTE, S. ; WATTS, J. D. ; AEBERSOLD, R.** Halogenated peptides as internal standards (H-PINS): Introduction of an MS-based internal standard set for liquid chromatography-mass spectrometry. *Mol. Cell. Proteomics,* 2009, vol. 8, 1934-1946
- **MORUZ, L. ; STAES, A. ; FOSTER, J. M. ; HATZOU, M. ; TIMMERMAN, E. ; MARTENS, L. ; KÄLL, L.** Chromatographic retention time prediction for posttranslationally modified peptides. *Proteomics,* 2012, vol. 12, 1151-1159
- **PRATT, J. M. ; SIMPSON, D. M. ; DOHERTY, M. K. ; RIVERS, J. ; GASKELL, S. J. ; BEYNON, R. J.** Multiplexed absolute quantification for proteomics using concatenated signature peptides encoded by QconCAT genes. *Nat. Protoc.,* 2006, vol. 1, 1029-1043
- **REIMER, J. ; SPICER, V. ; KROKHIN, O. V.** Application of modern reversed-phase peptide retention prediction algorithms to the Houghten and DeGraw dataset: Peptide helicity and its effect on prediction accuracy. *J. Chromatogr. A.,* 2012, vol. 1256, 160-168
- **SPICER, V. ; GRIGORYAN, M. ; GOTFRID, A. ; STANDING, K. G. ; KROKHIN, O. V.** Predicting retention time shifts associated with variation of the gradient slope in peptide RP-HPLC. *Anal. Chem.,* 2010, vol. 82, 9678-9685